# EUROPEAN PATENT APPLICATION

(11) **EP 1 498 493 A1**
(43) Date of publication of application: **19.01.2005**
(21) Application number: 03703371.9
(22) Date of filing: 25.02.2003
(51) Int. Cl.: C12Q 1/68, C12N 15/00, G01N 33/68, G01N 33/53, A61K 31/711, A61K 39/395, A61P 37/08

(54) **METHOD OF EXAMINING ALLERGIC DISEASE**

(30) Priority: 03.04.2002 JP 2002100908
(71) Applicant: Genox Research, Inc., Ibaraki 300-2635 (JP); Japan as represented by General Director of Agency of National Center for Child Health and Development, Tokyo 157-8535 (JP)
(72) Inventor: MATSUMOTO, Yoshiko, Genox Research, Inc., Kawasaki-shi, Kanagawa 216-0001 (JP); IMAI, Yukiho, Genox Research, Inc., Kawasaki-shi, Kanagawa 216-0001 (JP); YOSHIDA, Nei, Genox Research, Inc., Kawasaki-shi, Kanagawa 216-0001 (JP); OSHIDA, Tadahiro, Genox Research, Inc., Kawasaki-shi, Kanagawa 216-0001 (JP); SUGITA, Yuji, Genox Research, Inc., Kawasaki-shi, Kanagawa 216-0001 (JP); SAITO, Hirohisa, Setagaya-ku, Tokyo 154-8509 (JP)
(74) Representative: Grünecker, Kinkeldey, Stockmair & Schwanhäusser Anwaltssozietät
(86) International application number: PCT/JP2003/002047
(87) International publication number: WO 2003/083139

(57) **Abstract**

B1799 gene, whose expression level increases significantly in the patient group, was successfully identified by screening for a gene which shows difference in expression in blood collected from multiple healthy subjects and patients of allergic diseases employing the differential display method. The gene expression level is specifically high in T cells and increased due to T cell activation. This gene can be used in testing for allergic diseases and also in screening for therapeutic agents for allergic diseases.

SEQUENCE LISTING

<110> Genox Research, Inc.
JAPAN AS REPRESENTED BY GENERAL DIRECTOR OF AGENCY OF NATIONAL
CENTER FOR CHILD HEALTH AND DEVELOPMENT

<120> METHOD OF TESTING FOR ALLERGIC DISEASES

<130> G1-A0117P

<140>
<141>

<150> JP 2002-100908
<151> 2002-04-03

<160> 9

<170> PatentIn Ver. 2.1

<210> 1
<211> 5922
<212> DNA
<213> Homo sapiens

<220>

<221> CDS

<222> (348)..(4244)

<400> 1

gcggccgcgg ggaccctcgg cgtggtcctc tgaccctgca aacccgcgac ggaggaaggg 60

gaggtcctgc ccgaggcgcc agcccgagga ggaggatgcc catttaaccc gccctcgcct 120

gccgggcgct tgcctcggtg cccgccgccg gagcctccga gccgcgcccg tggaagtgct 180

gcatggggca gggctgctga agcgcggagt tcggggtcgc gccgctccca ggcaggcgcg 240

ggagcccggt gcggcagttg gcacagtttc ggcggcgcct tctgcgcggg agtggggggc 300

gcggtgcgcc cggccggcct ccgcggtgcc ctggtgaggc gagagtt atg gag ccg 356

Met Glu Pro

1

ccc agc tgc att cag gat gag ccg ttc ccg cac ccc ctg gag ccc gag 404

Pro Ser Cys Ile Gln Asp Glu Pro Phe Pro His Pro Leu Glu Pro Glu

    5                   10                  15

ccg ggc gtc tca gct cag ccc ggc ccc ggg aag cca agc gat aag cgg 452

Pro Gly Val Ser Ala Gln Pro Gly Pro Gly Lys Pro Ser Asp Lys Arg

20                  25                  30                  35

ttc cgg ctg tgg tac gtt ggg ggg tcg tgc ctg gac cac agg acc acg 500
Phe Arg Leu Trp Tyr Val Gly Gly Ser Cys Leu Asp His Arg Thr Thr
                40                  45                  50

ctg cct atg ctg ccc tgg ctc atg gcc gag atc cgc agg cgc agc cag 548
Leu Pro Met Leu Pro Trp Leu Met Ala Glu Ile Arg Arg Arg Ser Gln
            55                  60                  65

aag ccc gag gcg ggc ggc tgc ggg gcg ccg gcg gcc cga gag gtg atc 596
Lys Pro Glu Ala Gly Gly Cys Gly Ala Pro Ala Ala Arg Glu Val Ile
        70                  75                  80

ctg gtg ctc agc gcg ccc ttc ctg cgt tgc gtc ccc gcg ccg ggc gct 644
Leu Val Leu Ser Ala Pro Phe Leu Arg Cys Val Pro Ala Pro Gly Ala
    85                  90                  95

ggg gcc tcg ggg ggc act agt ccg tcg gcc acg cag ccc aac ccg gcg 692
Gly Ala Ser Gly Gly Thr Ser Pro Ser Ala Thr Gln Pro Asn Pro Ala
100                 105                 110                 115

gta ttc atc ttc gag cac aag gcg cag cat atc tcg cgc ttc atc cac 740
Val Phe Ile Phe Glu His Lys Ala Gln His Ile Ser Arg Phe Ile His
                120                 125                 130

aac agc cac gac ctc acc tac ttt gcc tac ctg atc aag gcg cag ccc 788
Asn Ser His Asp Leu Thr Tyr Phe Ala Tyr Leu Ile Lys Ala Gln Pro

            135                 140                 145

gac gac ccc gag tcg cag atg gcc tgc cac gtt ttc cgc gcc aca gac 836
Asp Asp Pro Glu Ser Gln Met Ala Cys His Val Phe Arg Ala Thr Asp
        150                 155                 160

ccc agc cag gtt cct gat gtt att agc agc ata agg caa tta tct aaa 884
Pro Ser Gln Val Pro Asp Val Ile Ser Ser Ile Arg Gln Leu Ser Lys
    165                 170                 175

gcg gcc atg aaa gag gat gcc aaa ccc agc aaa gat aat gag gac gcc 932
Ala Ala Met Lys Glu Asp Ala Lys Pro Ser Lys Asp Asn Glu Asp Ala
180                 185                 190                 195

ttt tac aac tct cag aag ttc gaa gtc ctg tac tgt gga aag gtg acc 980
Phe Tyr Asn Ser Gln Lys Phe Glu Val Leu Tyr Cys Gly Lys Val Thr
                200                 205                 210

gtg acc cac aag aag gcc ccc tca agc ctc atc gat gac tgc atg gag 1028
Val Thr His Lys Lys Ala Pro Ser Ser Leu Ile Asp Asp Cys Met Glu
            215                 220                 225

aag ttc agc ctg cac gaa cag cag cgc ctg aag atc caa ggc gag cag 1076
Lys Phe Ser Leu His Glu Gln Gln Arg Leu Lys Ile Gln Gly Glu Gln
        230                 235                 240

cgc ggt ccg gac cca gga gag gac ctg gct gac ttg gag gtg gtg gtg 1124
Arg Gly Pro Asp Pro Gly Glu Asp Leu Ala Asp Leu Glu Val Val Val
    245                 250                 255

ccc ggg tcc ccc gga gac tgc ctg ccg gag gag gct gac ggc acc gac 1172
Pro Gly Ser Pro Gly Asp Cys Leu Pro Glu Glu Ala Asp Gly Thr Asp
260                 265                 270                 275

acc cac ctt ggc tta cct gcc ggg gcc agc cag cct gcc ctg acc agc 1220
Thr His Leu Gly Leu Pro Ala Gly Ala Ser Gln Pro Ala Leu Thr Ser
                280                 285                 290

tct cgg gtc tgc ttc cct gag cgg att ttg gaa gat tct ggc ttt gat 1268
Ser Arg Val Cys Phe Pro Glu Arg Ile Leu Glu Asp Ser Gly Phe Asp
            295                 300                 305

gag cag cag gag ttt cgg tct cgg tgc agc agt gtc acc ggc gtg caa 1316
Glu Gln Gln Glu Phe Arg Ser Arg Cys Ser Ser Val Thr Gly Val Gln
        310                 315                 320

cgg aga gtt cac gag ggc agc cag aaa tcc cag ccg cga cgg aga cac 1364
Arg Arg Val His Glu Gly Ser Gln Lys Ser Gln Pro Arg Arg Arg His
    325                 330                 335

gcg agc gca ccc agt cac gtc cag ccc tcg gac tcg gag aag aac agg 1412
Ala Ser Ala Pro Ser His Val Gln Pro Ser Asp Ser Glu Lys Asn Arg

340                 345                 350                 355

acc atg ctc ttc cag gtt ggg cga ttt gag att aac ctt atc agt cca     1460
Thr Met Leu Phe Gln Val Gly Arg Phe Glu Ile Asn Leu Ile Ser Pro
                360                 365                 370

gac act aaa tca gtt gtg cta gaa aag aat ttt aaa gat atc tcc tct     1508
Asp Thr Lys Ser Val Val Leu Glu Lys Asn Phe Lys Asp Ile Ser Ser
            375                 380                 385

tgt tct cag ggt ata aag cat gtg gat cac ttt ggc ttt atc tgc cgg     1556
Cys Ser Gln Gly Ile Lys His Val Asp His Phe Gly Phe Ile Cys Arg
        390                 395                 400

gag tct cca gag cct gga ctt agc cag tat att tgt tat gta ttc cag     1604
Glu Ser Pro Glu Pro Gly Leu Ser Gln Tyr Ile Cys Tyr Val Phe Gln
    405                 410                 415

tgt gcc agc gaa tct ctg gtt gat gag gta atg ctg act ctg aaa cag     1652
Cys Ala Ser Glu Ser Leu Val Asp Glu Val Met Leu Thr Leu Lys Gln
420                 425                 430                 435

gcc ttc agt acg gcg gct gcc ctg cag agt gcc aag acg cag att aaa     1700
Ala Phe Ser Thr Ala Ala Ala Leu Gln Ser Ala Lys Thr Gln Ile Lys
                440                 445                 450

ctg tgt gag gcc tgc ccg atg cac tct ttg cat aag ctc tgt gaa agg 1748
Leu Cys Glu Ala Cys Pro Met His Ser Leu His Lys Leu Cys Glu Arg
            455                 460                 465

att gaa ggt ctc tac cca cca aga gcc aag ctg gtg ata cag agg cat 1796
Ile Glu Gly Leu Tyr Pro Pro Arg Ala Lys Leu Val Ile Gln Arg His
        470                 475                 480

ctc tca tca ctg aca gat aat gag caa gct gac atc ttt gaa aga gtt 1844
Leu Ser Ser Leu Thr Asp Asn Glu Gln Ala Asp Ile Phe Glu Arg Val
    485                 490                 495

cag aaa atg aag cca gtc agt gac cag gaa gaa aat gaa ctt gtg att 1892
Gln Lys Met Lys Pro Val Ser Asp Gln Glu Glu Asn Glu Leu Val Ile
500                 505                 510                 515

tta cac ctg agg cag ctg tgt gaa gcc aag cag aag aca cac gtg cac 1940
Leu His Leu Arg Gln Leu Cys Glu Ala Lys Gln Lys Thr His Val His
                520                 525                 530

atc ggg gaa ggc cct tct act att tca aat agt aca atc cca gaa aat 1988
Ile Gly Glu Gly Pro Ser Thr Ile Ser Asn Ser Thr Ile Pro Glu Asn
            535                 540                 545

gca aca agc agt gga agg ttc aaa ctt gac att ctg aaa aat aaa gct 2036
Ala Thr Ser Ser Gly Arg Phe Lys Leu Asp Ile Leu Lys Asn Lys Ala

        550                 555                 560

aag aga tcc tta act agc tcc ctg gaa aat atc ttc tca agg gga gct 2084
Lys Arg Ser Leu Thr Ser Ser Leu Glu Asn Ile Phe Ser Arg Gly Ala
    565                 570                 575

aac aga atg aga ggt cgg ctt gga agt gtg gac agt ttt gaa cgg tcc 2132
Asn Arg Met Arg Gly Arg Leu Gly Ser Val Asp Ser Phe Glu Arg Ser
580                 585                 590                 595

aac agt ctt gct tca gag aag gac tac tca cca ggg gat tct cca cca 2180
Asn Ser Leu Ala Ser Glu Lys Asp Tyr Ser Pro Gly Asp Ser Pro Pro
                600                 605                 610

ggg aca ccg cca gcg tcc cca ccg tcc tca gct tgg caa acg ttt ccc 2228
Gly Thr Pro Pro Ala Ser Pro Pro Ser Ser Ala Trp Gln Thr Phe Pro
            615                 620                 625

gaa gag gat tcc gac tcc ccg cag ttt cga aga cgg gca cac acg ttc 2276
Glu Glu Asp Ser Asp Ser Pro Gln Phe Arg Arg Arg Ala His Thr Phe
        630                 635                 640

agc cac cca cct tca agc aca aag aga aag ctg aat ttg cag gat ggg 2324
Ser His Pro Pro Ser Ser Thr Lys Arg Lys Leu Asn Leu Gln Asp Gly
    645                 650                 655

agg gct cag ggt gtg cgt tcc cct ctg ctg agg cag agc tcc agt gaa    2372
Arg Ala Gln Gly Val Arg Ser Pro Leu Leu Arg Gln Ser Ser Ser Glu
660                 665                 670                 675

cag tgc agc aat ctt tcg tca gtt cga cgc atg tac aag gag agt aat    2420
Gln Cys Ser Asn Leu Ser Ser Val Arg Arg Met Tyr Lys Glu Ser Asn
                680                 685                 690

tct tcc tcc agt ctt cca agt ctt cac act tcc ttc tct gcc cct tcc    2468
Ser Ser Ser Ser Leu Pro Ser Leu His Thr Ser Phe Ser Ala Pro Ser
            695                 700                 705

ttc act gcc ccc tct ttc ctg aaa agc ttt tac cag aat tca ggt aga    2516
Phe Thr Ala Pro Ser Phe Leu Lys Ser Phe Tyr Gln Asn Ser Gly Arg
        710                 715                 720

ctg tcc cca cag tat gaa aat gaa atc aga caa gac act gct tca gaa    2564
Leu Ser Pro Gln Tyr Glu Asn Glu Ile Arg Gln Asp Thr Ala Ser Glu
    725                 730                 735

tca agt gat gga gaa ggg aga aaa agg acc tca tct acc tgc agc aat    2612
Ser Ser Asp Gly Glu Gly Arg Lys Arg Thr Ser Ser Thr Cys Ser Asn
740                 745                 750                 755

gag tcc cta agt gtg gga gga acc tct gtc act cct cgc cgg atc tcc    2660
Glu Ser Leu Ser Val Gly Gly Thr Ser Val Thr Pro Arg Arg Ile Ser

                760                 765                 770

tgg cgg cag cgc att ttc ctc agg gtt gct tct ccc atg aac aaa tct 2708
Trp Arg Gln Arg Ile Phe Leu Arg Val Ala Ser Pro Met Asn Lys Ser
            775                 780                 785

ccc tca gca atg caa cag caa gat gga ttg gac agg aac gag ctg ctg 2756
Pro Ser Ala Met Gln Gln Gln Asp Gly Leu Asp Arg Asn Glu Leu Leu
        790                 795                 800

cca ctg tcc ccc ctc tct cca acc atg gag gag gaa ccg ctg gtt ata 2804
Pro Leu Ser Pro Leu Ser Pro Thr Met Glu Glu Glu Pro Leu Val Ile
    805                 810                 815

ttc ctg tct ggg gag gat gac cca gaa aag att gaa gaa aga aag aaa 2852
Phe Leu Ser Gly Glu Asp Asp Pro Glu Lys Ile Glu Glu Arg Lys Lys
820                 825                 830                 835

tca aaa gaa ctg agg agc ttg tgg aga aaa gct ata cac caa caa atc 2900
Ser Lys Glu Leu Arg Ser Leu Trp Arg Lys Ala Ile His Gln Gln Ile
                840                 845                 850

ttg tta ctt cga atg gaa aaa gaa aac cag aaa ctt gaa gga gca agc 2948
Leu Leu Leu Arg Met Glu Lys Glu Asn Gln Lys Leu Glu Gly Ala Ser
            855                 860                 865

aga gat gaa ctc cag tcc aga aaa gtt aaa tta gac tat gaa gaa gtt 2996
Arg Asp Glu Leu Gln Ser Arg Lys Val Lys Leu Asp Tyr Glu Glu Val
        870                 875                 880

ggt gca tgt cag aaa gag gtc tta ata act tgg gat aag aag ttg tta 3044
Gly Ala Cys Gln Lys Glu Val Leu Ile Thr Trp Asp Lys Lys Leu Leu
    885                 890                 895

aac tgc aga gct aaa atc aga tgt gat atg gaa gat att cat act ctt 3092
Asn Cys Arg Ala Lys Ile Arg Cys Asp Met Glu Asp Ile His Thr Leu
900                 905                 910                 915

ctt aaa gaa gga gtt ccc aaa agt cga cga gga gaa att tgg cag ttt 3140
Leu Lys Glu Gly Val Pro Lys Ser Arg Arg Gly Glu Ile Trp Gln Phe
                920                 925                 930

ctg gct tta cag tac cga ctc aga cac aga ttg cct aat aaa caa cag 3188
Leu Ala Leu Gln Tyr Arg Leu Arg His Arg Leu Pro Asn Lys Gln Gln
            935                 940                 945

cct cct gac ata tcc tat aag gaa ctt ttg aag cag ctc act gct cag 3236
Pro Pro Asp Ile Ser Tyr Lys Glu Leu Leu Lys Gln Leu Thr Ala Gln
        950                 955                 960

cag cat gcg att ctc gtg gat tta gga agg acg ttt cct act cac cct 3284
Gln His Ala Ile Leu Val Asp Leu Gly Arg Thr Phe Pro Thr His Pro

965                 970                 975

tac ttt tca gta cag ctt ggg cca gga cag ctg tca ctg ttt aac ctc     3332
Tyr Phe Ser Val Gln Leu Gly Pro Gly Gln Leu Ser Leu Phe Asn Leu
980                 985                 990                 995

ctg aaa gcc tat tct ttg ctg gac aaa gaa gtg gga tac tgt cag ggg     3380
Leu Lys Ala Tyr Ser Leu Leu Asp Lys Glu Val Gly Tyr Cys Gln Gly
                1000                1005                1010

atc agc ttt gtg gct gga gtc ctg ctt ctg cac atg agt gaa gag caa     3428
Ile Ser Phe Val Ala Gly Val Leu Leu Leu His Met Ser Glu Glu Gln
            1015                1020                1025

gcc ttt gaa atg ctg aaa ttc ctc atg tat gac ctc ggc ttc cgc aag     3476
Ala Phe Glu Met Leu Lys Phe Leu Met Tyr Asp Leu Gly Phe Arg Lys
        1030                1035                1040

cag tac aga cct gac atg atg tcg ctg cag att caa atg tac cag ctg     3524
Gln Tyr Arg Pro Asp Met Met Ser Leu Gln Ile Gln Met Tyr Gln Leu
    1045                1050                1055

tcc agg ctc ctt cat gac tat cac aga gat ctc tac aat cac ctt gaa     3572
Ser Arg Leu Leu His Asp Tyr His Arg Asp Leu Tyr Asn His Leu Glu
1060                1065                1070                1075

gaa aat gaa atc agc ccc agt ctt tat gct gcc ccc tgg ttc ctc aca 3620
Glu Asn Glu Ile Ser Pro Ser Leu Tyr Ala Ala Pro Trp Phe Leu Thr
                1080                1085                1090

ttg ttt gcc tct cag ttt tca tta gga ttt gta gcc aga gtt ttt gat 3668
Leu Phe Ala Ser Gln Phe Ser Leu Gly Phe Val Ala Arg Val Phe Asp
            1095                1100                1105

att att ttt ctt cag gga act gaa gtt ata ttc aag gtt gca ctc agc 3716
Ile Ile Phe Leu Gln Gly Thr Glu Val Ile Phe Lys Val Ala Leu Ser
        1110                1115                1120

cta ctg agc agc caa gag aca ctt ata atg gaa tgt gag agc ttt gaa 3764
Leu Leu Ser Ser Gln Glu Thr Leu Ile Met Glu Cys Glu Ser Phe Glu
    1125                1130                1135

aat att gtt gag ttt ctt aaa aac acg cta cct gat atg aat acc tct 3812
Asn Ile Val Glu Phe Leu Lys Asn Thr Leu Pro Asp Met Asn Thr Ser
1140                1145                1150                1155

gaa atg gaa aaa att att acc cag gtt ttt gag atg gat att tct aag 3860
Glu Met Glu Lys Ile Ile Thr Gln Val Phe Glu Met Asp Ile Ser Lys
                1160                1165                1170

cag ttg cat gcc tat gag gtg gaa tat cat gtg cta cag gat gag ctt 3908
Gln Leu His Ala Tyr Glu Val Glu Tyr His Val Leu Gln Asp Glu Leu

1175                 1180                 1185

cag gaa tct tca tat tcc tgt gag gat agt gaa act ttg gag aag ctg    3956
Gln Glu Ser Ser Tyr Ser Cys Glu Asp Ser Glu Thr Leu Glu Lys Leu
        1190                 1195                 1200

gag agg gcc aat agc caa ctg aaa aga caa aac atg gac ctc cta gaa    4004
Glu Arg Ala Asn Ser Gln Leu Lys Arg Gln Asn Met Asp Leu Leu Glu
    1205                 1210                 1215

aaa tta cag gta gct cat act aaa atc cag gcc ttg gaa tca aac ctg    4052
Lys Leu Gln Val Ala His Thr Lys Ile Gln Ala Leu Glu Ser Asn Leu
1220                 1225                 1230                 1235

gaa aat ctt ttg acg aga gag acc aaa atg aag tct tta atc cgg acc    4100
Glu Asn Leu Leu Thr Arg Glu Thr Lys Met Lys Ser Leu Ile Arg Thr
                1240                 1245                 1250

ctg gaa caa gaa aaa atg gct tat caa aag aca gtg gag caa ctc cgg    4148
Leu Glu Gln Glu Lys Met Ala Tyr Gln Lys Thr Val Glu Gln Leu Arg
            1255                 1260                 1265

aag ctg ctg ccc gcg gat gct cta gcc aat tgt gac ctg ttg ctg aga    4196
Lys Leu Leu Pro Ala Asp Ala Leu Ala Asn Cys Asp Leu Leu Leu Arg
        1270                 1275                 1280

gac cta aac tgc aac cct aac aac aaa gcc aag ata gga aat aag cca 4244
Asp Leu Asn Cys Asn Pro Asn Asn Lys Ala Lys Ile Gly Asn Lys Pro
    1285                1290                1295

taattgaaga ggcacggcct cagcagaaag tgctccttag aatactacag agaggaagag 4304

cctgcatgtc gctggcccaa ggctggaccc tgaagctgat ggaaccacct aatactggtg 4364

ctgagctcct agtcacagca ggtggacctc gtgctcatca gagcatgcca atcctaagcc 4424

attggacata tgtagactgg tttttgttgt tgctatgtac atataaatat atatataaaa 4484

tgaacatagt tcatgctttc agataaaatg agtagatgta tatttagatt aattttttta 4544

gtcagaactt catgaaatcc acaccaaagg aaaggtaaac tgaaatttcc cttggacata 4604

tgtgaaatct ttttgtcttt atagtgaaac aaagccagag catctttgta tattgcaata 4664

tacttgaaaa aaatgaatgt atttttttct ccaaagaaca gcatgtttca ctcaatggtg 4724

aaaaggtgga aacatttatg taactttatg tgtatctgtc ttgatatcta ctgacattgt 4784

ctatatgagg aaaatgatta ctggtcatgc tcctgtgagt tttttgggaa ggtagggtca 4844

tttctccctg cctgctttgt gccaactagc atgttgcatc tacatgcatt atgagtctgg 4904

ttaggcatta ctttaaacat acataaagag acagtaggac attgtggctg agtctaccca 4964

gctcaaggta aaggagaatg ttgctaattt tttagcaaac tagaccagca ttattactca 5024

aactaaaaat atcacacctg aaaaatttaa tttaggacct aaaatgtcta gattagcttt 5084

ctgctttttt tatttgaata actcattcag ttgtgaatga attcctcttt atttggtgcc 5144

acagtcacca aatgacaagg atttgccact ttcccaccaa attgtgagtg cttgtaattt 5204

aggtctctct accttaaatt cagtataagg aaacgtaatt atgattgatt ttttccaaag 5264

atgacaagct gtgttgaaat acattttttc ttttgaccaa ttgacagaat ctaataagct 5324

ttaataatct tcccctttta tgtgaaaagt tttgagaact gtgaaatgtt taggaacaaa 5384

ctgttgaaat ccattggaag ggaaaaaaga aagtggtacc agtgttacca gctcaactaa 5444

aacctgcaat tctgcatttc aactcttcac ttcctcagcc tacaaatagc tcattagatg 5504

acattcacgc atgctgggta taggcaagga aagtaatttt caaagtacat ttgcagttct 5564

ctttttcaga gatgattcta tgatagtgcc tctgaaagtt gatgcagcat tttgcctttt 5624

ccaaaaagta tttatcctca ctgctttttg cagtacttgt attttcacag atggattatc 5684

tggggtaatt ttcttcaaag ggagtttgtt atacacagtg aaaatgtatt atagagtaga 5744

atagtaaagc tctaggggtt tcagaaagct ttgatgaaca gatgacaaac atctgaaacc 5804

ccctccgcac tgttacccag tgtgtatata atgacttgtt atagctcagt gtgcccttga 5864

atccatacag tttcttaaaa gacaataaaa tcttattaat aaagttaatg taacttct 5922

<210> 2

<211> 1299

<212> PRT

<213> Homo sapiens

<400> 2

Met Glu Pro Pro Ser Cys Ile Gln Asp Glu Pro Phe Pro His Pro Leu
1               5                   10                  15

Glu Pro Glu Pro Gly Val Ser Ala Gln Pro Gly Pro Gly Lys Pro Ser
            20                  25                  30

Asp Lys Arg Phe Arg Leu Trp Tyr Val Gly Gly Ser Cys Leu Asp His
        35                  40                  45

Arg Thr Thr Leu Pro Met Leu Pro Trp Leu Met Ala Glu Ile Arg Arg
    50                  55                  60

Arg Ser Gln Lys Pro Glu Ala Gly Gly Cys Gly Ala Pro Ala Ala Arg
65                  70                  75                  80
Glu Val Ile Leu Val Leu Ser Ala Pro Phe Leu Arg Cys Val Pro Ala
                85                  90                  95
Pro Gly Ala Gly Ala Ser Gly Gly Thr Ser Pro Ser Ala Thr Gln Pro
            100                 105                 110
Asn Pro Ala Val Phe Ile Phe Glu His Lys Ala Gln His Ile Ser Arg
        115                 120                 125
Phe Ile His Asn Ser His Asp Leu Thr Tyr Phe Ala Tyr Leu Ile Lys
    130                 135                 140
Ala Gln Pro Asp Asp Pro Glu Ser Gln Met Ala Cys His Val Phe Arg
145                 150                 155                 160
Ala Thr Asp Pro Ser Gln Val Pro Asp Val Ile Ser Ser Ile Arg Gln
                165                 170                 175
Leu Ser Lys Ala Ala Met Lys Glu Asp Ala Lys Pro Ser Lys Asp Asn
            180                 185                 190
Glu Asp Ala Phe Tyr Asn Ser Gln Lys Phe Glu Val Leu Tyr Cys Gly

        195                 200                 205

Lys Val Thr Val Thr His Lys Lys Ala Pro Ser Ser Leu Ile Asp Asp

    210                 215                 220

Cys Met Glu Lys Phe Ser Leu His Glu Gln Gln Arg Leu Lys Ile Gln

225                 230                 235                 240

Gly Glu Gln Arg Gly Pro Asp Pro Gly Glu Asp Leu Ala Asp Leu Glu

                245                 250                 255

Val Val Val Pro Gly Ser Pro Gly Asp Cys Leu Pro Glu Glu Ala Asp

            260                 265                 270

Gly Thr Asp Thr His Leu Gly Leu Pro Ala Gly Ala Ser Gln Pro Ala

        275                 280                 285

Leu Thr Ser Ser Arg Val Cys Phe Pro Glu Arg Ile Leu Glu Asp Ser

    290                 295                 300

Gly Phe Asp Glu Gln Gln Glu Phe Arg Ser Arg Cys Ser Ser Val Thr

305                 310                 315                 320

Gly Val Gln Arg Arg Val His Glu Gly Ser Gln Lys Ser Gln Pro Arg

                325                 330                 335

Arg Arg His Ala Ser Ala Pro Ser His Val Gln Pro Ser Asp Ser Glu
            340                 345                 350

Lys Asn Arg Thr Met Leu Phe Gln Val Gly Arg Phe Glu Ile Asn Leu
        355                 360                 365

Ile Ser Pro Asp Thr Lys Ser Val Val Leu Glu Lys Asn Phe Lys Asp
    370                 375                 380

Ile Ser Ser Cys Ser Gln Gly Ile Lys His Val Asp His Phe Gly Phe
385                 390                 395                 400

Ile Cys Arg Glu Ser Pro Glu Pro Gly Leu Ser Gln Tyr Ile Cys Tyr
                405                 410                 415

Val Phe Gln Cys Ala Ser Glu Ser Leu Val Asp Glu Val Met Leu Thr
            420                 425                 430

Leu Lys Gln Ala Phe Ser Thr Ala Ala Ala Leu Gln Ser Ala Lys Thr
        435                 440                 445

Gln Ile Lys Leu Cys Glu Ala Cys Pro Met His Ser Leu His Lys Leu
    450                 455                 460

Cys Glu Arg Ile Glu Gly Leu Tyr Pro Pro Arg Ala Lys Leu Val Ile
465                 470                 475                 480

Gln Arg His Leu Ser Ser Leu Thr Asp Asn Glu Gln Ala Asp Ile Phe
                485                 490                 495

Glu Arg Val Gln Lys Met Lys Pro Val Ser Asp Gln Glu Glu Asn Glu
            500                 505                 510

Leu Val Ile Leu His Leu Arg Gln Leu Cys Glu Ala Lys Gln Lys Thr
        515                 520                 525

His Val His Ile Gly Glu Gly Pro Ser Thr Ile Ser Asn Ser Thr Ile
    530                 535                 540

Pro Glu Asn Ala Thr Ser Ser Gly Arg Phe Lys Leu Asp Ile Leu Lys
545                 550                 555                 560

Asn Lys Ala Lys Arg Ser Leu Thr Ser Ser Leu Glu Asn Ile Phe Ser
                565                 570                 575

Arg Gly Ala Asn Arg Met Arg Gly Arg Leu Gly Ser Val Asp Ser Phe
            580                 585                 590

Glu Arg Ser Asn Ser Leu Ala Ser Glu Lys Asp Tyr Ser Pro Gly Asp
        595                 600                 605

Ser Pro Pro Gly Thr Pro Pro Ala Ser Pro Pro Ser Ser Ala Trp Gln

    610                 615                 620

Thr Phe Pro Glu Glu Asp Ser Asp Ser Pro Gln Phe Arg Arg Arg Ala
625                 630                 635                 640

His Thr Phe Ser His Pro Pro Ser Ser Thr Lys Arg Lys Leu Asn Leu
                645                 650                 655

Gln Asp Gly Arg Ala Gln Gly Val Arg Ser Pro Leu Leu Arg Gln Ser
            660                 665                 670

Ser Ser Glu Gln Cys Ser Asn Leu Ser Ser Val Arg Arg Met Tyr Lys
        675                 680                 685

Glu Ser Asn Ser Ser Ser Ser Leu Pro Ser Leu His Thr Ser Phe Ser
    690                 695                 700

Ala Pro Ser Phe Thr Ala Pro Ser Phe Leu Lys Ser Phe Tyr Gln Asn
705                 710                 715                 720

Ser Gly Arg Leu Ser Pro Gln Tyr Glu Asn Glu Ile Arg Gln Asp Thr
                725                 730                 735

Ala Ser Glu Ser Ser Asp Gly Glu Gly Arg Lys Arg Thr Ser Ser Thr
            740                 745                 750

Cys Ser Asn Glu Ser Leu Ser Val Gly Gly Thr Ser Val Thr Pro Arg
        755                 760                 765
Arg Ile Ser Trp Arg Gln Arg Ile Phe Leu Arg Val Ala Ser Pro Met
    770                 775                 780
Asn Lys Ser Pro Ser Ala Met Gln Gln Gln Asp Gly Leu Asp Arg Asn
785                 790                 795                 800
Glu Leu Leu Pro Leu Ser Pro Leu Ser Pro Thr Met Glu Glu Glu Pro
                805                 810                 815
Leu Val Ile Phe Leu Ser Gly Glu Asp Asp Pro Glu Lys Ile Glu Glu
            820                 825                 830
Arg Lys Lys Ser Lys Glu Leu Arg Ser Leu Trp Arg Lys Ala Ile His
        835                 840                 845
Gln Gln Ile Leu Leu Leu Arg Met Glu Lys Glu Asn Gln Lys Leu Glu
    850                 855                 860
Gly Ala Ser Arg Asp Glu Leu Gln Ser Arg Lys Val Lys Leu Asp Tyr
865                 870                 875                 880
Glu Glu Val Gly Ala Cys Gln Lys Glu Val Leu Ile Thr Trp Asp Lys
                885                 890                 895

Lys Leu Leu Asn Cys Arg Ala Lys Ile Arg Cys Asp Met Glu Asp Ile
            900                 905                 910
His Thr Leu Leu Lys Glu Gly Val Pro Lys Ser Arg Arg Gly Glu Ile
        915                 920                 925
Trp Gln Phe Leu Ala Leu Gln Tyr Arg Leu Arg His Arg Leu Pro Asn
    930                 935                 940
Lys Gln Gln Pro Pro Asp Ile Ser Tyr Lys Glu Leu Leu Lys Gln Leu
945                 950                 955                 960
Thr Ala Gln Gln His Ala Ile Leu Val Asp Leu Gly Arg Thr Phe Pro
                965                 970                 975
Thr His Pro Tyr Phe Ser Val Gln Leu Gly Pro Gly Gln Leu Ser Leu
            980                 985                 990
Phe Asn Leu Leu Lys Ala Tyr Ser Leu Leu Asp Lys Glu Val Gly Tyr
        995                 1000                1005
Cys Gln Gly Ile Ser Phe Val Ala Gly Val Leu Leu Leu His Met Ser
    1010                1015                1020
Glu Glu Gln Ala Phe Glu Met Leu Lys Phe Leu Met Tyr Asp Leu Gly

1025                1030                1035                1040

Phe Arg Lys Gln Tyr Arg Pro Asp Met Met Ser Leu Gln Ile Gln Met

                1045                1050                1055

Tyr Gln Leu Ser Arg Leu Leu His Asp Tyr His Arg Asp Leu Tyr Asn

            1060                1065                1070

His Leu Glu Glu Asn Glu Ile Ser Pro Ser Leu Tyr Ala Ala Pro Trp

        1075                1080                1085

Phe Leu Thr Leu Phe Ala Ser Gln Phe Ser Leu Gly Phe Val Ala Arg

    1090                1095                1100

Val Phe Asp Ile Ile Phe Leu Gln Gly Thr Glu Val Ile Phe Lys Val

1105                1110                1115                1120

Ala Leu Ser Leu Leu Ser Ser Gln Glu Thr Leu Ile Met Glu Cys Glu

                1125                1130                1135

Ser Phe Glu Asn Ile Val Glu Phe Leu Lys Asn Thr Leu Pro Asp Met

            1140                1145                1150

Asn Thr Ser Glu Met Glu Lys Ile Ile Thr Gln Val Phe Glu Met Asp

        1155                1160                1165

Ile Ser Lys Gln Leu His Ala Tyr Glu Val Glu Tyr His Val Leu Gln
    1170                1175                1180

Asp Glu Leu Gln Glu Ser Ser Tyr Ser Cys Glu Asp Ser Glu Thr Leu
1185                1190                1195                1200

Glu Lys Leu Glu Arg Ala Asn Ser Gln Leu Lys Arg Gln Asn Met Asp
                1205                1210                1215

Leu Leu Glu Lys Leu Gln Val Ala His Thr Lys Ile Gln Ala Leu Glu
            1220                1225                1230

Ser Asn Leu Glu Asn Leu Leu Thr Arg Glu Thr Lys Met Lys Ser Leu
        1235                1240                1245

Ile Arg Thr Leu Glu Gln Glu Lys Met Ala Tyr Gln Lys Thr Val Glu
    1250                1255                1260

Gln Leu Arg Lys Leu Leu Pro Ala Asp Ala Leu Ala Asn Cys Asp Leu
1265                1270                1275                1280

Leu Leu Arg Asp Leu Asn Cys Asn Pro Asn Asn Lys Ala Lys Ile Gly
                1285                1290                1295

Asn Lys Pro

<210> 3

<211> 164

<212> DNA

<213> Homo sapiens

<400> 3

caaggtggat tgtgaattta tgctgtagcc aacttttagt tttgagaaac accataaaaa 60

caaattaagt tacctcattt actaggcgaa acaggcaagg ttaaggcata cacaaaaaga 120

agagttaatt cgtttgggtg gaaactcttt tgtttttcct ttca 164

<210> 4

<211> 486

<212> DNA

<213> Homo sapiens

<400> 4

aaagggctcc cttccgcttg tgtttcagga ctactcacca ggggattctc caccagggac 60

accgccagcg tccccaccgt cctcagcttg gcaaacgttt cccgaagagg attccgactc 120

cccgcagttt cgaagacggg cacacacgtt cagccaccca ccttcaagca caaagagaaa 180

gctgaatttg caggatggga gggctcaggg tgtgcgttcc cctctgctga ggcagagctc 240

cagtgaacag tgcaggtgag tctgaccctc tccagaatga gacctagatt ctcaaggcac 300

acagttttga ttactctgga gtcaaggtgg attgtgaatt tatgctgtag ccaactttta 360

gttttgagaa acaccataaa aacaaattaa gttacctcat ttactaggcg aaacaggcaa 420

ggttaaggca tacacaaaaa gaagagttaa ttcgtttggg tggaaactct tttgtttttc 480

ctttca 486

<210> 5

<211> 17

<212> DNA

<213> Artificial Sequence

<220>

<223> Description of Artificial Sequence: artificially

synthesized sequence

<400> 5

gttttttttt ttttttc 17

<210> 6

<211> 10

<212> DNA

<213> Artificial Sequence

<220>

<223> Description of Artificial Sequence: artificially

synthesized sequence

<400> 6
tctctggagt                                                              10

<210> 7
<211> 21
<212> DNA
<213> Artificial Sequence

<220>
<223> Description of Artificial Sequence: artificially
      synthesized sequence

<400> 7
aagacagtgg agcaactccg g                                                 21

<210> 8
<211> 21
<212> DNA
<213> Artificial Sequence

<220>
<223> Description of Artificial Sequence: artificially
      synthesized sequence

<400> 8

cagcaacagg tcacaattgg c                                                21

<210> 9

<211> 20

<212> DNA

<213> Artificial Sequence

<220>

<223> Description of Artificial Sequence: artificially

      synthesized sequence

<400> 9

agctgctgcc cgcggatgct                                                  20

## Description

### Technical Field

The present invention relates to methods of testing for an allergic disease and methods of screening for a therapeutic agent for an allergic disease.

### Background Art

Allergic diseases such as atopic dermatitis are considered to be multifactorial diseases. These diseases are caused by the interaction of many different genes whose expression is independently influenced by multiple environmental factors. Therefore, it is extremely difficult to identify a specific gene causing a specific disease. Furthermore, the expression of mutated or defective genes, or increased or decreased expression of specific genes is envisaged to be associated with allergic diseases. Thus, to reveal the role of gene expression in diseases, it is required to understand how a gene is involved at the onset of a disease and how external stimulus, such as drugs, alters gene expression.

History taking as well as confirmation of the patient's family history and own anamnesis, are important in general for recent diagnosis of allergic diseases. In addition, for allergy diagnosis based on more objective information, a method of testing patient' s blood samples and a method of observing patient's immune response to allergen are also performed. Examples of the former method are the allergen-specific IgE test, leukocyte histamine release test and lymphocyte blastogenesis test. The presence of allergen-specific IgE is proof of occurrence of the allergic reaction to the allergen. However, in some patients, allergen-specific IgE may not necessarily be detected. Furthermore, the IgE assay principle requires performing tests for all of the allergens necessary for diagnosis. The leukocyte histamine release test and the lymphocyte blastogenesis test are methods for observing the immune system reaction to allergens *in vitro.* These methods are complicated to perform.

Another known method is allergy diagnosis based on the immune response observed at the time when a patient is contacted with an allergen. Such a test includes the prick test, scratch test, patch test, intradermal reaction and induction test. These tests allow the direct diagnosis of patient's allergic reaction while they can be said to be highly invasive tests because patients are actually exposed to allergens.

In addition, test methods for proving the occurrence of allergic reaction caused by any allergen are also attempted. For example, a high serum IgE titer may indicate the occurrence of allergic reaction in the patient. The serum IgE titer corresponds to the total amount of allergen-specific IgE. Though it is easy to determine the total amount of IgE when any allergen is tested, the IgE titer may be reduced in some patients, such as, those with non-atopic bronchitis.

The number of eosinophils and eosinophil cationic protein (ECP) level are diagnostic items for delayed-type reaction following Type I allergy and allergic inflammatory reaction. The number of eosinophils is considered to reflect the progress of allergic symptoms. ECP, a protein contained in eosinophil granules, is also strongly activated in patients with an asthma attack. Indeed these diagnostic items identify allergy symptoms, but they are limited in their scope as diagnostic indicators.

Therefore, a marker (indicator) for an allergic disease that is not only less invasive to patients but also capable of readily providing information necessary for diagnosis would be useful. Since such markers are thought to be deeply involved in disease onset, they may be an important target in the control of allergic symptoms as well as in diagnosis.

### Disclosure of the Invention

An objective of the present invention is to provide an indicatormarker gene for testing an allergic disease. Another objective of the invention is to provide methods of testing for an allergic disease and methods of screening for a therapeutic agent for an allergic disease, both using the expression of the gene as a marker.

Based on a previously established technique, the "fluorescent differential display method (Fluorescent DD method)" (T. Ito et al. 1994, FEBS Lett. 351: 231-236), the present inventors developed a new DD system capable of analyzing T-cell RNA samples prepared from multiple human blood samples. Using Fluorescent DD method, the present inventors successfully isolated genes whose expression levels change in peripheral blood cells from patients with pollinosis. The genes that were isolated (along with the patent applications filed) are listed below:
Pollinosis-associated gene 373 (WO 00/65046),
Pollinosis-associated gene 419 (WO 00/65045),
Pollinosis-associated gene 513 (WO 00/65049),
Pollinosis-associated gene 581 (WO 00/65048),
Pollinosis-associated gene 795 (WO 00/65050),
Pollinosis-associated gene 627 (WO 00/65051),
Pollinosis-associated gene 441 (WO 00/73435),
Pollinosis-associated gene 465 (WO 00/73439), and
Pollinosis-associated gene 787 (WO 00/73440).

Similarly, genes whose expression levels in peripheral blood differ between patients of allergic diseases and healthy subjects were searched using differential display method. As a result, gene B1153 whose expression level significantly rose in the patient group was discovered and filed as a patent application (WO 02/50269).

The present inventors used the Fluorescent DD method to isolate genes whose expression level is altered in an allergic disease-specific manner. Specifically, first, the present inventors collected blood from normal healthy subjects and patients with allergic diseases (atopic dermatitis and allergic asthma), isolated T cells from the blood, and via the Fluorescent DD method screened for genes whose expression levels differ between the normal healthy subject group and the patient group. As a result, the present inventors succeeded in isolating a gene, "B1799," that showed significantly higher expression levels in the patient group. The isolated gene was the same as KIAA0603 (GenBank Accession No. AB011175). Genomic sequence information and expression data are available for the mouse homologue of KIAA0603 (GenBank Accession No. AB011175; Genomics 2002 Feb; 79(2):154-61 Candidate genes required for embryonic development: a comparative analysis of distal mouse chromosome 14 and human chromosome 13q22. Kurihara LJ, Semenova E, Miller W, Ingram RS, Guan XJ, Tilghman SM. Howard Hughes Medical Institute and Department of Molecular Biology, Princeton University, Princeton, New Jersey 08544, USA). According to the report, sequences adjacent to the gene encoding Endothelin B receptor were determined, and the mouse homologue was shown as one of the genes comprised in this adjacent region. Furthermore, since deletion of this region in mice led to embryonic death, these genes were suggested to be related to this incident. No information relating to immunity and allergy is included in the report. The isolated human B1799 gene encodes a protein consisting of 1299 amino acids. This protein has the characteristic of a GTPase activating protein, which indicated that it is involved in cell differentiation and cell cycle control. The expression level of the B1799 gene was higher in atopic dermatitis patients compared to normal healthy subjects, especially higher in patients with moderate symptom. Analysis of the expression of the gene in various types of leukocytes revealed that the expression was high particularly in T cells, more particularlyin memory T cells (cells with the phenotypes of CD4⁺ and CD45RO⁺). Furthermore, the present inventors discovered from the result of in vitro stimulation test that expression of B1799 mRNA is induced during the activated cell death of T cells. Specifically, B1799 mRNA expression was induced under conditions that activated the T cells by stimulus via T cell receptors or stimulus such as calcium ionophores (ionomycins). This relation of B1799 gene expression with the activation of the T cells verifies the involvement of the gene in allergic diseases. Thus, the inventors found that testing for allergic diseases and screening of candidate compounds serving as therapeutic agents for allergic diseases are possible by using the expression level of the gene as a marker, and completed this invention.

The present invention relates to methods of testing for allergic diseases and methods of screening for therapeutic agents for allergic diseases based on the expression level of a marker gene, B1799 gene, which shows a high expression in allergic disease patients. Specifically, the present invention relates to the following testing methods for allergic diseases, kits therefor, and methods of screening for therapeutic agents for allergic diseases:
(1) A method of testing for an allergic disease, the method comprising the steps of:
   (a) measuring the expression level of a marker gene in a biological sample from a test subject;
   (b) comparing the expression level with that of the marker gene in a biological sample from a healthy subject; and
   (c) judging the test subject to have an allergic disease when the expression level of the marker gene in the biological sample from the test subject is found to be significantly elevated,
   wherein the marker gene is B1799 gene.
(2) The testing method according to (1), wherein the allergic disease is atopic dermatitis.
(3) The testing method according to (1), wherein the gene expression level is measured via cDNA PCR.
(4) The testing method according to (1), wherein the gene expression level is measured by detecting a protein encoded by the gene.
(5) The testing method according to (1), wherein the biological sample contains peripheral blood T cells.
(6) A reagent for diagnosis of an allergic disease, said reagent comprising a polynucleotide that comprises at least 15 continuous nucleotide sequence of B1799 gene or a complementary sequence thereof;
(7) A reagent for testing for an allergic disease, said reagent comprising an antibody that binds to a polypeptide consisting of the amino acid sequence encoded by B1799 gene;
(8) A method for screening a therapeutic agent for an allergic disease, wherein said method comprises the steps of:
   (a) contacting a candidate compound with a cell expressing a marker gene;
   (b) measuring the expression level of said marker gene; and
   (c) selecting a compound which decreases the expression level of the marker gene as compared to a control where said candidate compound has not been contacted,
   wherein the marker gene is B1799 gene.
(9) The method according to (8), wherein the cell is T cell.
(10) A method of screening for a therapeutic agent for an allergic disease, the method comprising the steps of:
   (a) administering a candidate compound to a test animal;
   (b) measuring the expression level of a marker gene in leukocytes of the test animal; and
   (c) selecting a compound which decreases the expression level of the marker gene compared to a control where the candidate compound has not been administered,
   wherein the marker gene is B1799 gene.
(11) A method of screening for a therapeutic agent for an allergic disease, the method comprising the steps of:
   (a) contacting a candidate compound with cells containing a reporter gene linked under the control of transcriptional regulatory region of a marker gene;
   (b) measuring the expression level of the reporter gene; and
   (c) selecting a compound which decreases the expression level of the reporter gene compared to a control where the candidate compound has not been contacted,
   wherein the marker gene is B1799 gene.
(12) A method of screening for a therapeutic agent for an allergic disease, the method comprising the steps of:
   (a) contacting a candidate compound with a protein encoded by a marker gene or a gene functionally equivalent thereto;
   (b) measuring the activity of the protein; and
   (c) selecting a compound which decreases the activity of the protein compared to a control where the candidate compound has not been contacted,
   wherein the marker gene is B1799 gene.
(13) A therapeutic agent for an allergic disease comprising as the main ingredient a compound obtainable by the screening method according to any one of (8), (10), (11), and (12).
(14) A therapeutic agent for an allergic disease, which comprises as a main ingredient an antisense DNA that contains a sequence complementary to a sequence comprising at least 15 continuous nucleotides of the sense strand sequence of a marker gene,
   wherein the marker gene is B1799 gene.
(15) A therapeutic agent for an allergic disease, which comprises as a main ingredient an antibody which binds to a protein encoded by a marker gene, wherein the marker gene is B1799 gene.
(16) An animal model of allergic disease, wherein said animal is a transgenic nonhuman vertebrate, in which the expression level of a marker gene, or a gene functionally equivalent thereto, has been increased in T cells, wherein the marker gene is B1799 gene.
(17) A kit for screening for a therapeutic agent for an allergic disease, the kit comprising a polynucleotide comprising at least 15 continuous nucleotide sequence of a marker gene or its complementary sequence,wherein the marker gene is B1799 gene.
(18) A kit for screening for a therapeutic agent for an allergic disease, the kit comprising an antibody which binds to a polypeptide consisting of an amino acid sequence encoded by a marker gene and cells expressing the marker gene, wherein the marker gene is B1799 gene.

The present invention also relates to a method of treating an allergic disease, which comprises the step of administering a compound of any one of the following (A) to (C). In addition, the present invention relates to the use of a compound of any one of the following (A) to (C) for producing a therapeutic agent for an allergic disease:
(A) a compound obtainable by the screening method according to any one of (8), (10), (11), and (12) above;
(B) an antisense DNA having a sequence complementary to a sequence comprising at least 15 continuous nucleotides of the sense strand sequence of B1799 gene; and
(C) an antibody which binds to a protein encoded by B1799 gene.

Furthermore, the present invention relates to a method of producing an allergic disease animal model, the method comprising the step of increasing the expression level of B1799 gene or a gene functionally equivalent thereto in T cells in a nonhuman vertebrate. In addition, the present invention relates to the use of a transgenic nonhuman vertebrate, in which the expression level of B1799 gene or a gene functionally equivalent thereto has been increased, as an allergic disease animal model.

The present invention relates to methods of testing for allergic diseases using the expression level of B1799 gene as a marker in leukocytes of a test subject. According to the present invention, B1799 gene was demonstrated to show a higher expression level in a patient group with allergic diseases as compared to the group of healthy subjects. Therefore, allergic diseases can be tested using the expression level of B1799 gene as a marker. Herein, B1799 gene is referred to as "the marker gene." The term "allergic disease" used herein is a general term for diseases involving allergic reactions. More specifically, this term is defined as a disease for which an allergen is identified, a strong correlation between exposure to the allergen and the onset of the pathological change is demonstrated, and the pathological change has been proven to have an immunological mechanism. Herein, an immunological mechanism means that leukocytes show an immune response to allergen stimulation. Examples of allergens are mite antigens, pollen antigens, etc.

Representative allergic diseases include atopic dermatitis, allergic rhinitis, pollinosis, bronchial asthma and insect allergy. Allergic diathesis is a genetic factor that is inherited from allergic parents to children. Familial allergic diseases are also called atopic diseases, and their causative factor that can be inherited is atopic diathesis. Among allergic diseases, asthma is a general term for diseases accompanied with respiratory organ symptoms.

As used herein, the term "B1799 gene" refers to human B1799 gene identified in Examples and a gene located in the same locus or a gene located in a homologous locus in another organism. For example, in the present invention, B1799 gene includes an arbitrary allele in the same locus as or homologous locus to that of the human B1799 gene identified in Examples. The nucleotide sequence of the transcription product of the human B1799 gene identified in Examples and the amino acid sequence of a protein encoded by the gene is shown in SEQ ID Nos: 1 and 2, respectively. Specifically, in the present invention, B1799 gene includes the human B1799 gene (SEQ ID NO:1) identified in Examples, its polymorphic variants (including SNPs), mutants, splicing variants, and homologues from other organisms. Since the expressional control for these genes are presumed to be substantially identical or similar to that of the human B1799 gene shown in SEQ ID NO:1, tests for allergic diseases according to the present invention can be carried out by detecting their expression.

Specifically, in the present invention, B1799 gene is substantially identical to an endogenous gene that comprises the following nucleic acid. The endogenous gene refers to an artificially unmodified gene contained in an organism in nature, and a gene that comprises the same nucleotide sequence is substantially identical to the endogenous gene. Furthermore, herein the term "gene" refers to a transcription product or nucleic acid (e.g., DNA, RNA, etc.) encoding the same.
(a) A nucleic acid that comprises a nucleotide sequence selected from the nucleotide sequence of SEQ ID NO:1.
(b) A nucleic acid comprising a nucleotide sequence of a coding sequence of SEQ ID NO:1, which contains one or more nucleotide substitutions, deletions and/or insertions.
(c) A nucleic acid encoding at least 15 continuous amino acids of the amino acid sequence of SEQ ID NO:2.
(d) A nucleic acid which encodes a protein comprising the amino acid sequence of SEQ ID NO:2 containing one or more amino acid substitutions, deletions and/or insertions.
(e) A nucleic acid which hybridizes under a stringent condition to a nucleic acid containing at least 50 continuous nucleotides of SEQ ID NO:1 but no nucleotide sequence derived from any sequence other than SEQ ID NO:1.

The nucleic acid according to (a) includes those containing preferably at least 40, more preferably 60, 100, 200, 500, and 1,000 or more continuous nucleotides of the nucleotide sequence of SEQ ID NO:1 (more preferably the coding region of SEQ ID NO:1;nt 348 to nt 4244), and most preferably its 5922 nucleotides (i.e., the whole length). Such nucleic acids include polymorphic variants, mutants, and splicing variants of the gene primarily within the same species. Examples of the nucleic acid according to (a) are those containing the nucleotide sequences of at least 30, more preferably 35, 40, 45, and 50 or more continuous nucleotides of the nucleotide sequence of SEQ ID NO:1. Nucleotide sequence is preferably selected from the coding region of SEQ ID NO:1 (nt 348 to nt 4244) , and any desirable numbers of nucleotide sequences may be selected. In the case of selecting a plurality of nucleotide sequences, it is desirable to select nucleic acids comprising the continuous nucleotide sequences at 2 or more, preferably 3, 4, and 5 or more different sites so that the nucleic acids do not overlap.

The nucleic acid according to (b) includes those containing the nucleotide sequences of the coding region of SEQ ID NO:1 (nt 348 to nt 4244 in SEQ ID NO:1) in which preferably 15% or less, more preferably 10%, 8%, 5%, and 1% or less of the total nucleotides is substituted, deleted, and/or inserted. Such nucleic acids may include counterpart genes of other close relative species, polymorphic variants, mutants, and splicing variants thereof. Such nucleic acids are those containing nucleotide sequences having the sequence identity of preferably 85% or more, more preferably 90%, 92%, 95%, and 99% or more to the coding region set forth in SEQ ID NO: 1.

Nucleic acid or amino acid sequence identity can be determined using, for example, a BLAST program (Altschul, S. F. et al., 1990, J. Mol. Biol. 215: 403-410). More specifically, nucleotide sequence identity is determined using the blastn program, while amino acid sequence identity using blastp program, and sequence identity search is conducted, for example, using default parameters with all filters containing Low complexity off at the BLAST website of the National Center for Biotechnology Information (NCBI) (Altschul, S. F. et al. (1993) Nature Genet. 3: 266-272; Madden, T. L. et al. (1996) Meth. Enzymol. 266: 131-141; Altschul, S. F. et al. (1997) Nucleic Acids Res. 25: 3389-3402; Zhang, J. and Madden, T. L. (1997) Genome Res. 7: 649-656). For example, using the blast2sequences program (Tatiana, A. et al. (1999) FEMS Microbiol Lett. 174: 247-250) for comparing two sequences, the two sequences can be aligned to determine the sequence identity. In this case, gaps are similarly treated as mismatches to calculate the identity value for the entire coding region of SEQ ID NO:1.

The nucleic acid according to (c) includes those encoding preferably at least 20, more preferably 30, 50, 100, 300, and 500 or more continuous amino acids of the amino acid sequence of SEQ ID NO:2, and most preferably its 1299 amino acids (i.e., the whole length). Such nucleic acids include, similarly as in the above-described (a), polymorphic variants, mutants, and splicing variants of genes primarily within the same species of organism. Furthermore, the nucleic acid according to (c) includes those containing partial nucleotide sequences encoding the amino acid sequence of at least 8, more preferably 10, 15, 20, and 25 or more continuous amino acids of SEQ ID NO:2 at 2 or more, preferably 3 or more, more preferably 4 and 5 or more sites so that each nucleic acids do not overlap.

The nucleic acid according to (d) includes those encoding the amino acid sequence of SEQ ID NO:2 in which preferably 15% or less, more preferably 10%, 8%, 5%, and 1% or less of the whole number of its amino acid residues are substituted, deleted, and/or inserted. These nucleic acids may contain untranslated sequences. Such nucleic acids include a counterpart gene of other close relative species, polymorphic variants, mutants, and splicing variants. Such nucleic acids are those encoding proteins comprising amino acid sequences having the sequence identity of preferably 85% or more, more preferably 90%, 92%, 95%, and 99% or more to the amino acid sequence of SEQ ID NO:2. Treating gaps similar to mismatches, the identity value relative to the whole amino acid sequence of SEQ ID NO:2 is calculated. Amino acid sequence identity can be determined according to the above-described method.

The nucleic acid according to (e) includes those hybridizing to nucleic acids comprising preferably at least 80, more preferably 100, 120, and 200 or more continuous nucleotides of SEQ ID NO:1 (more preferably its coding region; nt 348 to nt 4244) , and substantially containing no other nucleotide sequence than that of SEQ ID NO:1 (more preferably its coding region; nt 348 to nt 4244) under the stringent condition. Such nucleic acids may be those hybridizing under the stringent condition to probes that have been prepared, for example, using the nucleic acid containing the nucleotide sequence of SEQ ID NO:1 as a template.. Probes of 50 to several hundreds of nucleotides long can be synthesized, for example, by the DNA synthesis method, or by the random prime method, nick translation method and PCR method.

The stringent condition for the nucleic acid according to (e) is that hybridization is carried out in a hybridization solution preferably containing NaCl in the range of about 0.5 to about 0.9 M at 60°C, preferably at 62°C, more preferably at 65°C, and then washing is performed at the same temperature as for hybridization in 1 x SSC, preferably in 0.5 x SSC, more preferably in 0.2 x SSC, still more preferably in 0.1 x SSC for 1 h. In this case, the temperature conditions of hybridization and washing that greatly influence the stringency can be adjusted according to the melting temperature (Tm) of the probe, which depends on the ratio of constituent nucleotides of the probe to the base pairs to which the probe hybridizes, the length of the probe, and the composition (concentrations of salts and formamide) of a hybridization solution. Considering these conditions, those skilled in the art can empirically set up the appropriate condition to confer the equivalent stringency. As a hybridization solution, for example, 4 x SSC, preferably ExpressHyb™ Hybridization Solution (Clontech) etc. may be used.

B1799 gene preferably encodes a GTPase activating protein (GAP). GAP is a protein binding to the GTP binding protein. The GTPase activity of the GTP binding protein is enhanced by the interaction with GAP (Scheffzek, K. et al., Trends Biochem. Sci., 1998, 23(7): 257-262). The GTP binding protein that plays an important role in signal transduction, is activated when GTP binds to it and inactivated when the bound GTP is converted to GDP. The GTP binding protein itself has GTPase activity, and GAP enhances its GTPase activity. GAP controls signal transduction by assisting conversion of the GTP binding protein to the inactivated form. Furthermore, B1799 gene of the present invention preferably encodes a protein having a phosphotyrosine interaction domain (PID) that is involved in the binding between proteins at the phosphotyrosine binding domain and a TBC domain. The PID domain is conserved in proteins interacting with phophotyrosine, and is found in a group of proteins involved in various biological processes comprising signal transduction mediated by cell surface receptors or occurring during protein transport (Bork, P. and Margolis, B., 1995, Cell 80 (5) : 693-694). The TBC domain was discovered in Tbc1 (Richardson, P.M. and Zon, L.I., 1995, Oncogene 11 (6) : 1139-1148) , and is commonly seen in oncogene tre-2 and yeast cell cycle regulating factor BUB2 and cdc16. The TBC domain exists in Gyp6 and Gyp7, and is considered to be a domain widely distributed in GAPs of Rab-like GTPases (Neuwald, A.F., 1997, Trends Biochem. Sci. 22(7): 243-244).

In a preferred embodiment of the present invention, B1799 gene is a nucleic acid encoding a transcription product, which is transcribed from a promoter regulating the transcription of a genomic DNA that encodes mRNA comprising the nucleic acid of SEQ ID NO: 1 in a native human cell or from the counterpart of the promoter in other species.

Specifically, methods of testing for allergic diseases according to the present invention include the steps of: (a) measuring the expression level of a marker gene (i.e., B1799 gene) in a biological sample from a test subject; (b) comparing the expression level with that in a corresponding sample from a healthy subject; and (c) judging the test subject as being affected with an allergic disease if the expression level of the marker gene in the biological sample of the test subject is enhanced compared to that in the healthy subject. Since B1799 gene can be used as a marker in the present invention, B1799 gene is herein also called simply a marker gene. As described above, B1799 gene of the present invention includes, beside the human gene, homologues of other species. Therefore, unless otherwise stated, B1799 gene of species other than human means endogenous homologues in those species. If the expression level of B1799 gene in a test subject is higher than that in a healthy subject, the test subject is judged to have an allergic disease. Alternatively, a standard value may be set in advance based on the expression level of B1799 gene in a healthy subject, and the expression level in a test subject can be compared to the standard value. Methods to set a standard value and acceptable range based on the measured value of the marker gene are well known in the art. For example, a range of ± 2 S.D. can be used as the acceptable range. If the expression level of the marker gene in the test subject is within the acceptable range, the test subject is suggested to be normal with respect to allergic diseases; and if the expression level is over the range, the subject is judged to have an allergic disease.

The present testing methods can be conducted by using not only B1799 gene alone but its combination with other genes that serve as a marker for allergic diseases. The accuracy of the test can be improved by detecting the expression of multiple genes in combination. Generally, allergic disease patients form a heterogeneous population, and thus, a more accurate diagnosis is enabled by using multiple genes as markers.

In this invention, expression of the marker gene includes both transcription and translation. That is, the expression level of the marker gene can be the level of mRNA or protein corresponding to the gene. Therefore, the method of testing for allergic diseases of the present invention may be performed based on comparing the level of mRNA corresponding to the marker gene, or the level of the protein encoded by the gene. The measurement of the mRNA level can be carried out according to known genetic analysis methods. Specifically, one can use, for example, a hybridization technique using the nucleic acids that hybridize to the marker gene as probes, or a gene amplification technique using DNAs that hybridize to that gene as primers.

The probes or primers used for the testing of the present invention can be designed based on the nucleotide sequence of the marker gene. For example, the nucleotide sequence of the human B1799 gene identified in Example and the amino acid sequence encoded by the gene are described in SEQ ID NOs: 1 and 2, respectively. In general, genes of higher animals are often accompanied by polymorphism. Many molecules produce isoforms comprising different amino acid sequences from each other during the splicing process. Any genes can be used as the marker gene of the present invention, even though they differ from the above-mentioned gene in the nucleotide sequence due to polymorphism, alternative splicing, and such.

As a primer or probe can be used a polynucleotide comprising at least 15, preferably at least 20, more preferably at least 30, and much more preferably at least 35 continuous nucleotides of the nucleotide sequence of B1799 gene or its complementary sequence. Herein, the term "complementary sequence" refers to the nucleotide sequence of one strand of a double stranded polynucleotide, which is composed of A: T (or A: U) and G:C base pairs, to the other strand. Furthermore, such polynucleotides may comprise nucleotide sequences other than a part of the nucleotide sequence of B1799 or its complementary sequence. In addition, polynucleotides that are at least 70%, preferably 80% or more, more preferably 90% or more, and much more preferably 95% or more identical to the nucleotide sequence of at least 20, preferably 30, more preferably 35, and much more preferably 40 continuous nucleotides of B1799 gene or its complementary sequence can be used as reagents for testing allergic diseases of the present invention. The degree of homology between nucleotide sequences can be determined by an algorithm, such as BLAST. The detection of the marker gene is enabled by specific hybridization of the above-mentioned polynucleotides to the gene. A specific hybridization can be confirmed if there is no significant cross-hybridization with DNA and/or RNA encoding other genes under the above-mentioned stringent conditions.

Such polynucleotides are useful as probes to detect the marker gene, and as primers to amplify the marker gene. When used as a primer, those polynucleotides have a chain length of usually 15 bp to 100 bp, preferably 15 bp to 35 bp. When used as a probe, DNAs comprising the whole sequence of the marker gene (or its complementary strand), or its partial sequence that contains at least 15-bp, are used. When used as a primer, its 3' region must be complementary to the marker gene, while the 5' region can be linked to a restriction enzyme-recognition sequence or tag.

"Polynucleotides" as used in the present invention may be either DNA or RNA. These polynucleotides may be either synthetic or naturally-occurring. Also, DNA used as a probe for hybridization is usually labeled. Examples of labeling methods are described below. Herein, the term "oligonucleotide" refers to polynucleotides with relatively low degree of polymerization. A chain length of an oligonucleotide is, for example, within 100 nucleotides. Oligonucleotides are included in polynucleotides. The labeling methods are as follows:
· nick translation labeling using DNA polymerase I;
· end labeling using polynucleotide kinase;
· fill-in end labeling using Klenow fragment (Berger SL, Kimmel AR. (1987) Guide to Molecular Cloning Techniques, Methods in Enzymology, Academic Press; Hames BD, Higgins SJ (1985) Genes Probes: A Practical Approach. IRL Press; Sambrook J, Fritsch EF, Maniatis T. (1989) Molecular Cloning: a Laboratory Manual, 2nd Edn. Cold Spring Harbor Laboratory Press);
·transcription labeling using RNA polymerase (Melton DA, Krieg PA, Rebagkiati MR, Maniatis T, Zinn K, Green MR. (1984) Nucleic Acid Res., 12, 7035-7056); and
· non-radioisotopic labeling of DNA by incorporating modified nucleotides (Kricka LJ. (1992) Nonisotopic DNA Probing Techniques. Academic Press).

For testing for allergic diseases using hybridization techniques, for example, Northern hybridization, dot blot hybridization, or DNA microarray technique may be used. Furthermore, gene amplification techniques, such as RT-PCR method may be used. In RT-PCR, RNA is extracted from a biological sample, cDNA is prepared by its reverse transcription, and PCR is conducted using the obtained cDNA as the template to determine the gene expression level. By using the PCR amplification monitoring method during the gene amplification step in RT-PCR, one can achieve more quantitative analysis for the gene expression of the present invention.

In the PCR gene amplification-monitoring method, the detection target (DNA or reverse transcript of RNA) is hybridized to probes that are dual-labeled at both ends with different fluorescent dyes whose fluorescence cancels each other out. When the PCR proceeds and Taq polymerase degrades the probe as a result of its 5'-3' exonuclease activity, the two fluorescent dyes become distant from each other and the fluorescence is detected. The fluorescence is detected in real time. By simultaneously measuring a standard sample in which the copy number of the target is known, it is possible to determine the copy number of the target in the subject sample with the cycle number where PCR amplification is linear (Holland P. M. et al., 1991, Proc. Natl. Acad. Sci. USA 88: 7276-7280; Livak K. J. et al., 1995, PCR Methods and Applications 4(6): 357-362; Heid C. A. et al., 1996, Genome Research 6: 986-994; Gibson E. M. U. et al., 1996, Genome Research 6: 995-1001). For the PCR amplification-monitoring method, for example, ABI PRISM7700 (PE Biosystems) may be used.

Methods of testing for allergic diseases of the present invention can also be carried out by detecting a protein encoded by the marker gene. Hereinafter, a protein encoded by the marker gene is described as a marker protein. For example, for such test methods, Western blotting, immunoprecipitation method, and ELISA method may be employed using an antibody that binds to the marker protein.

Antibodies that bind to the marker protein used in the detection may be produced by techniques well known to those skilled in the art. Antibodies used in the present invention may be polyclonal or monoclonal antibodies (Milstein C. et al., 1983, Nature 305 (5934): 537-40). For example, polyclonal antibodies against the marker protein may be produced by collecting blood from mammals sensitized with the antigen, and separating the serum from this blood using known methods. As polyclonal antibodies, serum containing polyclonal antibodies may be used. If desired, a fraction containing polyclonal antibody can be further isolated from this serum. Alternatively, monoclonal antibodies may be obtained by isolating immune cells from mammals sensitized with the antigen, fusing these cells with myeloma cells, and such, cloning the obtained hybridomas, and collecting the antibodies from the culture of the hybridomas.

For detecting a marker protein, these antibodies may be appropriately labeled. Alternatively, instead of labeling the antibody, a substance that specifically binds to the antibody, for example, protein A or protein G, may be labeled to indirectly detect the marker protein. Specifically, one example of a detection method is ELISA.

A protein or its partial peptide used as an antigen may be obtained, for example, by inserting a gene encoding the protein or its portion into an expression vector, introducing the vector into an appropriate host cell to produce a transformant, culturing the transformant to express the recombinant protein, and purifying the expressed recombinant protein from the culture or the culture supernatant. Alternatively, oligopeptides consisting of a partial amino acid sequence of the protein can be chemically synthesized to be used as the immunogen.

Furthermore, methods of testing for allergic diseases of the present invention can be conducted using the activity of marker protein in leukocytes as a marker. The activity of marker protein refers to the biological activity possessed by the protein. More specifically, activity such as GAP activity, i.e., activation of the GTP binding protein, can be mentioned as the activity of the marker protein of the present invention. Alternatively, its interaction with the GTP binding protein can be used as the index of the activity. Detection of the activity of the marker protein can be preformed based on conventional methods. More specifically, the GAP activity can be determined by measuring the GTPase activity of a specific GTP binding protein on which this protein acts. For example, Gyp6 protein specifically acts on Ypt6 protein, a GTP binding protein, and enhances its GTPase activity. Therefore, by detecting the increase in GTPase activity of Ypt6 protein, the activity of Gyp6 protein can be measured (Strom M. et al., 1993, Nature 361(6414): 736-739). Thus, by measuring the activity of a specific GTP binding protein on which the marker protein B1799 of the present invention acts, the activity of the marker protein can be measured.

Normally, in testing methods of this invention, a biological sample from a subject is used as a test sample. A biological sample such as peripheral blood T cells can be used. Leukocytes used for the test may be purified or partially purified. Preferably, leukocytes prepared from peripheral blood, more preferably T-cells, of subjects are used as the test sample. T-cells can be prepared from peripheral blood by known methods. Specifically, for example, heparinized blood is collected, diluted, fractionated by centrifugation using Ficoll to separate PBMC. The separated PBMC may be used as it is as the sample for tests for allergic diseases of the present invention. PBMC contains lymphocytes and monocytes. As shown in Examples, among these blood cells, the marker gene is highly expressed in T cells. The expression level of the marker gene can be measured without great influence of cells other than T cells. Direct analysis of not a purified T-cell fraction but a lymphocyte fraction as a test sample enables a convenient bed-side test. Alternatively, T cells can be isolated by allowing them to be specifically adsorbed by microbeads to which the anti-CD3 antibody has been immobilized.

The intracellular mRNA of the marker gene or the marker protein can be measured in disintegrated blood cells. It is also possible to measure the marker protein in blood. For the preparation of T cell lysate and extraction of mRNA, kits such as an RNeasy Mini™ (Qiagen) and ISOGEN™ (Nippon Gene) on the market may be conveniently used.

The measured value of expression level of the marker gene in test biological samples can be corrected by known methods. The changes in the gene expression level in the biological samples can be compared using the corrected values. The measured value of the expression level of the genes that are to be used as markers in the present invention may be corrected based on the measured value of the expression level of the genes (such as housekeeping genes) that are expressed in the biological samples and do not largely fluctuate in their expression levels regardless of the condition of the cell. Examples of such genes are β-actin gene and glyceraldehyde 3-phosphate dehydrogenase (GAPDH) gene.

According to test methods of the present invention, the expression level of the marker gene in leukocytes from a test subject and the expression level in the corresponding leukocytes from a healthy subj ect are measured. It is examined whether the expression level in the leukocytes of the test subject is increased compared to that in the healthy subject. The increase in the expression level of the marker gene in the leukocytes from the test subject is correlated with allergic diseases.

The testings for an allergic disease according to the present invention include, for example, those as described below. Even a patient who cannot be judged as having an allergic disease by the ordinary testing in spite of showing allergic disease-like symptoms can be easily judged as an allergic disease patient by testings of this invention. More specifically, the elevation of the marker gene expression in patients with symptoms suspect of an allergic disease indicates that the symptoms would probably be those of an allergic disease. Allergic disease-like symptoms are exemplified by dermatitis (itching, flare), rhinitis (nasal congestion, running nose, sneeze) , and asthma (stridor, dyspnea). Methods of testing for allergic diseases according to the present invention include tests for determining whether such allergic disease-like symptomsare caused by allergic reaction. Although these symptoms are also observed in xeroderma, cold syndrome (coryza) , bronchitis, and such, it is possible to determine whether these symptoms are caused by allergic reaction or not, according to test methods of the present invention. In addition, methods of testing for allergic diseases of the present invention include tests to determine whether a subject has allergic diathesis or not. Specifically, such a test is conducted for a subject without noticeable symptom, and the subject is determined to have allergic diathesis if an increase in the expression level of the marker gene is detected. For treatment of an allergic disease-like symptom, it is a very important process to determine whether allergic reaction is the cause of the symptom or not. Testing methods of this invention can provide extremely important information for identifying the cause of diseases. Alternatively, even without referring to information of symptoms, allergic disease patients can be screened by testing samples and by screening samples that highly express the marker gene.

In addition, the testing methods is useful to judge whether allergic symptoms are getting ameliorated or not. The expression level of the marker gene of this invention increases in T cells which are contained in PBMC of patients with an allergic disease. T cells play a central role in immune response. Therefore, the decreased expression level of the marker gene in patients diagnosed as having an allergic disease indicates amelioration of allergic symptoms, and the increased expression level indicates that the allergic symptoms would be in progress.

Furthermore, the expression of the marker gene B1799 has been revealed to be high in T cells among leukocytes, especially in T cells having the phenotypes of CD4⁺ and CD45RO⁺ (i.e., memory T cells). Therefore, the marker gene can be used as a marker for these T cells. Moreover, the expression of the marker gene is significantly induced in activated cell death of T cells. In addition, the expression is also induced by activation stimuli by a stimulant such as ionomycin. Thus, since the expression of the marker gene reflects the state of T cell activation, the activation level of T cells may be determined by measuring the expression of the marker gene in T cells.

The present invention also relates to the use of a transgenic nonhuman animal, in which the expression level of the marker gene B1799 or a gene functionally equivalent thereto is elevated in T cells, as an allergic disease model animal. Allergic disease animal models are useful in clarifying changes in vivo in allergy. Furthermore, allergic disease animal models of this invention are useful in the assessment of therapeutic agents for an allergic disease.

The present invention demonstrated the elevated expression level of the marker gene in T cells. Therefore, animals in which the expression level of the B1799 gene or a gene functionally equivalent thereto is artificially elevated in T cells can be utilized as the allergic disease model animal. Herein, transgenic animals refer to animals genetically modified by insertion, substitution, and/or deletion of one or more nucleotides in their genomic DNAs. Such allergic disease animal models are useful in clarifying changes *in vivo* in allergy. Furthermore, allergic disease animal models of this invention are useful in assessing and screening for therapeutic agents for an allergic disease. In this case, the increased expression level in T cells includes the increased expression level of the marker gene in the whole blood cells. That is, the expression level elevation of the marker gene includes that in not only T cells but also blood cells as a whole or the whole body.

Genes functionally equivalent to the B1799 gene used in the present invention refer to those encoding proteins having the activity equivalent to that of the protein (marker protein) encoded by the marker gene B1799. Such genes include artificial variants of the human B1799 gene or its counterparts from other organisms. For example, in the case of inserting a gene into an expression vector, deletion of nucleotides corresponding to several amino acid residues at the N- or C-terminus, and addition of nucleotides corresponding to a tag peptide sequence or other amino acid sequence derived from the expression vector are frequently performed. The resulting proteins are different in the amino acid sequence from the endogenous protein in natural cells, but their activities are substantially equivalent to that of the endogenous protein. Herein, genes encoding such proteins are referred to as genes functionally equivalent to the endogenous gene.

One example of the activities of the marker protein used in this invention is the GAP activity. For example, a gene encoding the human B1799 protein of SEQ ID NO:2 in which one or more amino acids are deleted, inserted, and/or substituted and which maintains the GAP activity, can be used to prepare the transgenic model animal of this invention. Such modified proteins should have a homology of, for example, 90% or more, preferably 95% or more and, still more preferably 99% or more to the amino acid sequence of the human B1799 protein. Furthermore, genes which comprises nucleotide sequences hybridizing to the sequence of SEQ ID NO:1 under stringent conditions , and encode proteins having the GAP activity, can also be used as genes functionally equivalent to the B1799 gene.

The animal models of this invention can be used as a model for an allergic disease, for example, in a method comprising the steps of: (a) detecting a phenotype of the animal model, and (b) correlating the difference of the phenotype of said animal model from the corresponding phenotype of a control animal, whose expression level of the marker gene in T cells is lower compared to that of said animal model, with an allergic disease. Examples of the control animals include non-transgenic animal having the same genetic background as that of said animal model and a transgenic animal in which an empty vector has been introduced. The phenotype may be any desired phenotypes including allergic symptoms such as dermatitis, rhinitis, and asthma, or the activation of immunocytes or changes in gene expression.

In the present invention, it is highly significant to assess roles of the marker gene B1799 and effects of drugs targeting this gene using transgenic animals in which the expression level of the marker gene is elevated in T cells, as the allergic disease animal model. Furthermore, the allergic disease animal models according to the present invention are useful not only in screening for pharmaceutical agents for the treatment or prophylaxis of an allergic disease as described below but also in elucidating the mechanism of allergic diseases, and, furthermore, testing the safety of screened compounds. For example, if allergic disease model animals according to this invention either develop clinical manifestations of atopic dermatitis or allergic asthma, or show changes in measured values associated with any allergic diseases, it is possible to construct a system for screening for compounds that can cure the allergic conditions.

Herein, the elevation of the expression level refers to any of the states that: a target gene introduced as an exogenous gene is allowed to be expressed; transcription of a gene inherent in the host and/or its translation into protein are/is increased; and, decomposition of the protein that is a translation product is suppressed. The gene expression level can be confirmed by, for example, quantitative PCR as described in Examples. Furthermore, the expression level or activity of the translation product protein can be confirmed by comparing it with that in a normal state.

A typical transgenic animal is an animal which has been transfected with a gene of interest and allowed to express the gene. In another type of transgenic animals, the half-life of mRNA may be extended by removing a sequence from the untranslated region (UTR) of mRNA, which renders RNA unstable. Furthermore, in another type of transgenic animals, a mutation is introduced into the coding region of the marker gene to increase its activity or modify the amino acid sequence of the gene product protein so as to be hardly decomposed. Examples of mutation in the amino acid sequence are substitution, deletion, insertion, or addition of amino acid residues. In addition, mutation in the transcriptional regulatory region of the marker gene also enables to enhance the expression of the gene.

Methods for obtaining transgenic animals targeting a specific gene are well-known in the art. That is, a transgenic animal can be obtained by a method where the gene and ovum are mixed and treated with calcium phosphate; a method where the gene is directly introduced into pronuclei of oocyte under a phase contrast microscope using a micropipette (microinj ection method, U. S. Patent No. 4,873,191) ; a method where the gene is introduced into embryonic stem cells (ES cells), etc. Furthermore, other methods include a method where ovum is infected with a gene-inserted retroviral vector and a sperm vector method where a gene is introduced into ovum mediated by sperm. The sperm vector method is a gene recombination technique for introducing an exogenous gene and performed by allowing an exogenous gene to adhere to a sperm or to be incorporated in a sperm by the electroporation method or such and fertilizing ovum with the sperm (M. Lavitranoet, et al. , Cell, 57, 717, 1989).

Transgenic animals used as allergic disease animal models of the present invention can be produced using all the vertebrates except for humans. More specifically, transgenic animals in which various genes have been introduced and their expression levels are modified are produced using vertebrates such as mice, rats, rabbits, miniature pigs, goats, sheep, monkeys, and cattle.

Furthermore, the present invention relates to methods of screening for therapeutic agents for allergic diseases. According to the present invention, the marker gene shows a statistically significant increase in its expression level in the case of allergic diseases. Therefore, it is possible to obtain a therapeutic agent for an allergic disease by selecting a compound capable of reducing the expression level of this gene. Herein, compounds that reduce the expression level of a gene refer to those having inhibitory effects on any steps of the transcription or translation of the gene, stability of a transcript (mRNA) or a translation product (protein) , and the expression of a gene function such as protein activity.

Methods of screening for a therapeutic agent for allergic diseases of the present invention can be carried out either in vivo or in vitro. An in vivo screening method can be carried out, by the steps of:
(a) administering a candidate compound to a test animal;
(b) measuring the expression level of the marker gene in leukocytes of the test animal; and
(c) selecting the compound that reduces the expression level of the marker gene, compared to a control without administration of the compound.

As a test animal in the screening method of the present invention, any desirable animal that expresses a marker gene of the present invention may be used. Specifically, such animals include, for example, mice, rats, guinea pigs, rabbits, cats, pigs, miniature pigs, goats, cattle, sheep, and monkeys. Preferably animals with elevated expression of the marker gene are used. Furthermore, by using animals that exhibit allergic symptoms, the effect of candidate compounds on the symptoms can be evaluated.

The effect of a candidate drug compound can be detected by administering the candidate drug compound to a test animal and monitoring the action of the compound on the expression level of the marker gene in leukocytes of the animal. The change in the expression level of the marker gene in leukocytes of the test animal can be monitored by the same method as the above-mentioned test methods of the present invention. Based on the detection result, candidate drug compounds for allergic diseases can be screened by selecting candidate drug compounds that reduce the expression level of the marker gene.

More specifically, the screening methods of the present invention can be carried out by collecting leukocytes from a test animal to which a candidate compound has been given, and comparing the expression level of the marker gene with that in corresponding leukocytes collected from an animal without administration of the candidate compound. Leukocytes used for the tests may be purified or crude samples and include PBMC and purified T cells. Preferably, purified T-cells are used. Methods for collecting and preparing these biological samples are known in the art.

These screening methods enable the selection of drugs relating to the expression of the marker gene in various ways. Specifically, for example, drug candidate compounds having the following activity can be found:
reduce the transcriptional activity of the marker gene;
reduce the translation level from the transcript of the marker gene;
inhibit the activity of translation product of the marker gene; and
reduce the stability of the transcript of the marker gene or accelerate its decomposition.

The above-described screening methods of the present invention also include a method comprising the step of stimulating test animals with an allergen before and/or after the administration of a candidate compound. When the allergen stimulation is performed prior to the administration of a candidate compound, it is possible to detect the activity of a candidate compound that inhibits immune response occurring after the allergen stimulation. Compounds obtainable by this screening method are expected to have therapeutic effects on an allergic disease. When allergen stimulation is conducted after the candidate compound administration, it is possible to detect the activity of a candidate compound that suppresses initiation of immune response occurring by the allergen stimulation. Compounds obtainable by this screening method are expected to have prophylactic effects on an allergic disease. An allergen usable in the screening methods of this invention includes allergenic substances known in the art. More specifically, allergenic substances well-known in the art include mite, house dust, plant pollen, proteins derived from diverse foods, etc. These allergens may be derived from the nature or synthesized by gene recombination technique and the like method. Furthermore, allergens may be protein fragments. Methods for preparing a purified allergen are also well-known in the art.

For example, as a model closely resembling human atopic dermatitis, a spontaneous dermatitis model using NC/Nga mouse has been reported. Administration of the mite antigen (5 µg/ear) into the auricle of this mouse 8 times in total at 2 to 3 days intervals enables the induction of symptoms that closely resemble human atopic dermatitis after two weeks. Screening methods according to this invention can be conducted by administering a candidate compound to this system and monitoring changes in the expression level of a marker gene of this invention.

*In vitro* screening can be performed, for example, by a method in which a candidate compound is contacted with cells expressing the marker gene to select compounds that reduce the expression level of the marker gene. The method may be carried out, for example, by the following steps of:
(a) contacting a candidate compound with cells that express the marker gene;
(b) measuring the expression level of the marker gene; and
(c) selecting the compound that reduces the expression level of the marker gene, compared to a control with which the compound has not been contacted.

Peripheral blood leukocytes and cell lines derived from leukocytes can be used as marker gene-expressing cells. Specifically, T cells can be exemplified as the leukocytes. T cell lines include Molt4 cells and Jurkat cells. Human acute leukemia T cell line Jurkat (ATCC Number TIB-152) can be obtained from ATCC.

In screening methods of the present invention, first a candidate compound is added to the cell. Then, the expression level of the marker gene in the cell is measured to select the compound that reduces the expression level of the gene compared to that of a cell which has not been contacted with the candidate compound (control).

In screening methods of the present invention, expression levels of the marker gene can be compared by detecting the mRNA level transcribed from the gene or protein level encoded by the gene. When comparing the expression level using mRNA, an mRNA sample is prepared from the cells as described above. When comparing the expression level using protein, a protein sample is prepared from cells. Detection of mRNA and protein can be performed by known methods as described above.

Furthermore, based on the disclosure of this invention, it is possible to obtain the transcriptional regulatory region for the marker gene of this invention to construct a reporter assay system. A reporter assay system means a assay system for screening for compounds that regulate the transcriptional activity of a transcriptional regulatory region using the expression level of a reporter gene localized downstream of the transcriptional regulatory region as a marker. Specifically, such screening comprises the steps of:
(a) contacting a candidate compound with cells comprising a reporter gene that is linked to function under the control of a transcriptional regulatory region of the marker gene;
(b) measuring the expression level of the reporter gene; and
(c) selecting the compound that decreases the expression level of the reporter gene, compared to a control with which the candidate compound has not been contacted.

The DNA region of about 0.5kb to about 5kb upstream from the transcription initiation point can be used as a transcriptional regulatory region. Transcriptional regulatory regions may include known transcriptional factor binding sequences, transcriptional regulating elements, and furthermore, CAAT box and TATA box, which are normally seen in the promoter region. Examples of the reporter genes include chloramphenicol acetyltransferase (CAT) gene, luciferase gene, growth hormone genes. The expression level of these reporter genes may be measured by detecting the activity of the expressed protein. Methods for detecting the activity of the proteins are well known in the art. A candidate compound that decreases the expression level of the reporter gene compared to that in a cell without having been contacted with the candidate compound, is selected.

The genomic sequence encoding the human B1799 gene (GenBank Ac. No. AL139230) has been revealed. Based on the sequence, the transcription initiation point and the upstream promoter sequence can be determined. A reporter construct can be prepared by amplifying the promoter region of B1799 gene utilizing PCR and such based on the sequence information, and linking a reporter gene downstream of the promoter region. Specifically, for example, a transcriptional regulatory region used for the screening of the present invention can be obtained as follows. First, screening is performed by a method that uses PCR or hybridization based on the nucleotide sequence of the marker gene disclosed in the present invention or the sequence of the promoter region of B1799 gene, to obtain a genomic DNA clone containing the cDNA sequence from a human genome DNA library, such as BAC library and YAC library. Based on the obtained genomic DNA sequence, the transcriptional regulatory region is obtained from upstream of the cDNA disclosed in the present invention. A reporter construct is constructed by cloning the obtained transcriptional regulatory region so that it is positioned upstream of the reporter gene. The resulting reporter construct is transformed into a cultured cell strain to prepare a transformant for screening. By contacting candidate compounds with this transformant, compounds that regulate the expression of reporter genes can be screened.

Furthermore, cells containing a reporter gene linked so as to function under the control of the transcriptional regulatory region of the marker gene may be cells of so-called knockin animal. Knockin animal refers to a transgenic animal in which an exogenous gene has been inserted into the protein-coding region of the endogenous target gene. Since this knocked-in exogenous gene is inserted downstream of the transcriptional regulatory region of the target gene, it is expressed under a similar expression control to that for the endogenous target gene. It is possible to carry out the screening using the knockin animal or cells obtained from it using the reporter gene as a knockin gene. A screening method using a knockin animal comprises the steps of: (a) administering a candidate compound to an animal in which a reporter gene has been knocked-in to the marker gene site, (b) measuring the expression level of the reporter gene, and (c) selecting a compound capable of reducing the expression level of the reporter gene compared to a control in which the candidate compound has not been administered. For example, the expression level of the reporter gene in leukocytes, more preferably in T cells of the knockin animal to which a candidate compound is administered or not administered, are measured to select a compound capable of reducing the expression level. Furthermore, a screening method using cells from the knockin animal comprises the steps of: (a) contacting a candidate compound with cells from an animal in which a reporter gene has been knocked-in to the marker gene site, (b) measuring the expression level of the reporter gene, and (c) selecting a compound capable of reducing the expression level of the reporter gene compared to that in a control in which the candidate compound has not been contacted. For example, the expression level of the reporter gene in leukocytes, more preferably in T cells obtained from a knockin animal are measured in the presence or absence of a candidate compound to select a compound capable of reducing the expression level. These screenings are included in the screening method of the present invention. In the screening using an individual animal in particular, it is preferable to perform the screening using a knockin heterozygote in which one allele is left intact so as to express the marker gene, while the reporter gene has been knocked in the other allele, to prevent the complete deletion of the function intrinsic to the marker gene.

Alternatively, screening based on the activity of the marker protein can be employed as the in vitro screening method of the present invention. More specifically, the present invention relates to a method of screening for therapeutic agents for allergic diseases comprising the steps of:
(a) contacting a candidate compound with a protein encoded by the marker gene of the present invention or a gene functionally equivalent thereto;
(b) measuring the activity of the protein; and
(c) selecting the compound that reduces the activity of the protein as compared to a control with which the candidate compound has not been contacted.

Herein, genes functionally equivalent to the marker gene include endogenous B1799 gene that has been artificially modified as described above.

Such screening can be conducted, for example, by allowing host cells to exogenously express the marker gene and measuring the activity of the marker protein. In this case, the marker gene may be inserted into an expression vector, and the resulting recombinant vector is introduced into appropriate hosts such as mammalian cells. The vectors can be introduced into the host by, for example, biological, physical, or chemical methods. Examples of the biological method are a method using viral vectors, a method using a specific receptor, and cell fusion method (via HVJ (Sendai virus) , polyethylene glycol (PEG) method, electric cell fusion method, and microcell-mediated chromosome transfer). Examples of the physical method are a microinjection method, electroporation method, and a method using the gene particle gun (gene gun). Examples of the chemical method are a calcium phosphate precipitation method, liposome method, DEAE-dextran method, protoplast method, red cell ghost method, red cell membrane ghost method, and microcapsule method.

Activities of the marker protein measured in the screening methods include, for example, binding activity to GTP binding proteins or GAP activity. Furthermore, for example, the marker protein is suggested to be related to the regulation of intracellular signal transduction in T cells, and the regulation of differentiation or proliferation of T cells. Therefore, using these activities as an index, compounds having an activity to inhibit these activities may be screened. Such compounds obtained by the method repress the function of the marker protein. As a result, allergic immune response can be suppressed through the inhibition of the marker protein activity that is induced in the T cells.

The polynucleotide, antibody, cell line, or animal model required for the various screening methods of the present invention may be previously combined into a kit. More specifically, a kit is composed of, for example, a cell (cultured cell, animal, or such) expressing the marker gene, and a reagent for measuring the expression level of the marker gene. Reagents used for measuring the expression level of the marker gene include, for example, oligonucleotides comprising at least 15 continuous nucleotides of the nucleotide sequence of the marker gene or the complementary sequence thereof. Alternatively, antibodies binding to polypeptides comprising the amino acid sequence of the marker protein can be used as the reagent. In the kit may be packaged a substrate compound used for the detection of a label, medium and a container for cell culturing, positive and negative standard samples, and furthermore, a manual describing how to use the kit.

Candidate compounds used in these screening methods include compound preparations, such as immunosuppressors, synthesized by existing chemical methods, compound preparations synthesized by combinatorial chemistry, mixtures of multiple compounds such as extracts from animal or plant tissues, or microbial cultures, and their purified preparations.

Compounds selected by screening methods of the present invention are useful as a therapeutic agent for an allergic disease. Alternatively, antisense DNA capable of inhibiting the expression of the marker gene in this invention is useful for this purpose. Such antisense DNAs are those comprising sequences complementary to the sequences containing preferably at least 20, more preferably 25, 30, 40, 50, and 100 or more continuous nucleotides of the sense strand of the marker gene of this invention. The antisense region may be antisense to any region of transcripts (any transcripts before and after the processing, including the intermediary product) of the marker gene. One example of such region is that containing the translation initiation codon. Furthermore, an antibody binding to the protein encoded by the marker gene used in the present invention is useful as a therapeutic agent for an allergic disease. Preferable antibodies are those binding to the domain interacting with a GTP binding protein, such as, antibodies to the TBC domain (the amino acid region from position 965 to 1184 of SEQ ID NO: 2 or homologous region thereto), specifically antibodies that identify as an epitope a peptide comprising the Arg residue (e.g., the position 973 Arg residue of SEQ ID NO:2) necessary as the GTPase activating site, are preferred. Antibodies against a phosphotyrosine interaction domain (PID) (the amino acid region from position 167 to 237 or from 415 to 487 of SEQ ID NO: 2, or homologous region thereto) are also preferred. Therapeutic agents for an allergic disease according to the present invention contain a compound selected by the screening methods, the antisense DNA, or the antibody as at least one main ingredient, and can be prepared by mixing the ingredient with a physiologically acceptable carrier, excipient, diluent, etc. Therapeutic agents for an allergic disease according to this invention can be administered orally or parenterally to ameliorate allergic symptoms.

For an oral drug, the dosage form can be granules, powder, tablets, capsules, solution, emulsion, suspension, etc. Examples of injections are subcutaneous, intramuscular and peritoneal injections.

Furthermore, in the case where a compound to be administered is a protein, the therapeutic effect can be achieved by introducing a gene encoding the protein into the living body using a gene therapy technique. A technique for treating a disease by introducing a gene into the living body that encodes a protein having a therapeutic effect is well-known in the art.

Alternatively, an antisense DNA can be directly administered. In that case, cell membrane permeability or stability of the DNA can be elevated by, for example, 5' end and/or 3' end modification. Alternatively, the antisense DNA may be incorporated downstream of an appropriate promoter sequence to be administered as an antisense RNA expression vector. When this expression vector is introduced into T cells of an allergic disease patient, the expression level of the marker gene can be reduced due to the expression of antisense of the gene, thereby achieving a therapeutic effect on an allergic disease. For introducing the expression vector into T cells, methods performed either *in vivo* or *ex vivo* are known.

Although the dosage of a therapeutic agent for an allergic disease according to the invention may vary depending on the age, sex, body weight, and symptoms of a patient; therapeutic effects; methods for administration; treatment duration; types of active ingredient contained in a pharmaceutical composition or such, it can be usually administered in the range of 0.1 mg to 500 mg, preferably 0.5 mg to 20 mg per dose for an adult. However, since the dosage varies according to various conditions, an amount less than the above-described dosage may be sufficient in some cases, and a dosage exceeding the above-described range may be required in other cases.

### Brief Description of the Drawings

Fig. 1 shows the amount of expression of B1799 in atopic dermatitis patient samples.
Fig. 2 shows the amount of expression of B1799 in leukocytes in peripheral blood.
Fig. 3 shows the amount of expression of B1799 in T cell subgroups derived from peripheral blood.
Fig. 4 shows the (a) the change in the rate of dead cells during T cell activated cell death, and (b) the change in the expression of B1799 during T cell activated cell death.
Fig. 5 shows the change in the expression of B1799 due to T cell stimulation.
Fig. 6 shows the structure of B1799.
Fig. 7 shows the predicted tertiary structure model of B1799 protein. Arg343 of Gyp1p and Arg973 of B1799 are shown by arrows.

### Best Mode for Carrying Out the Invention

The present invention will be explained in detail below with reference to examples, but it is not to be construed as being limited thereto.

### [Example 1]

### Identification of B1799

The Differential Display (DD) method (Liang and Pardee, Science, 1992, 257: 967-971; T. Ito et al., 1994, FEBS Lett. 351: 231-236) discovered a 164bp DNA fragment (clone B1799-01) (SEQ ID NO: 3; except for primer sequence) that is expressed much stronger in T cells of atopic dermatitis patients and allergic asthma patients compared to that of healthy subjects. For the DD analysis to detect the fragment, GT15C (SEQ ID NO: 5) and AG00189 (TCTCTGGAGT; SEQ ID NO: 6) were used as anchor primer and arbitrary primer, respectively. The gene from which the fragment was derived was designated as B1799.

### [Example 2]

### Cloning of B1799

PCR cloning was performed based on the 5'-RACE method using the DD sequence and as a template a human leukocyte cDNA library to obtain a DNA fragment of a full length of 486bp (SEQ ID NO: 4). BLAST search on a public database revealed that the sequence at the position 27 to 255 of this nucleotide sequence was 100% identical to the gene KIAA0603 (GenBank Accession No. AB011175) that was identified by Kazusa DNA Research Institute. KIAA0603 is a cDNA of 5922bp that encodes a protein consisting of 1299 amino acid residues starting with methionine in its ORF. The homologous sequence region was within the ORF region, i.e., from nt 2152 to nt 2380, of KIAA0603 gene. Furthermore, 486bp sequence was completely identical with the nt 128071 to nt 127586 region (reverse strand) of GenBank Accession No. AL162571 that is a genomic sequence of chromosome 13.

### [Example 3]

### Expression quantification of B1799 by quantitative PCR

The DD sequence was determined to be derived from the immature mRNA intron region of KIAA0603 gene. Assuming that B1799 is identical to KIAA0603, KIAA0603 gene was analyzed. The following primers and probes for mRNA quantification were designed within the ORF of KIAA0603 gene and used for expression quantification. TQ1799orf is a TaqMan probe used in gene expression quantification by TaqMan method, whose 5' end and 3' end are fluorescent labeled with FAM (6-carboxyfluorescein) and TAMRA (6-carboxy-methyl-rhodamine), respectively. Using ABI-PRISM 7700, DNA amplification was detected at real time.

1799orf-f: AAGACAGTGGAGCAACTCCGG (SEQ ID NO: 7)

1799orf-r: CAGCAACAGGTCACAATTGGC (SEQ ID NO: 8)

TQ1799orf: AGCTGCTGCCCGCGGATGCT (SEQ ID NO: 9)

### [Example 4]

### Relevance to pathology

The expression of B1799 gene was measured using RNAs prepared from T cells of atopic dermatitis patients as described in Example 3. The results are shown in Table 1. According to a t-test of the subjects divided into two groups, i.e., healthy subject group and atopic dermatitis patient group, the expression level was significantly higher (p<0.05) in the patient group.

**[Table 1]**

| Expression level of B1799 in atopic dermatitis patients | | | | | |
|---|---|---|---|---|---|
| | Pathology | B1799(copy/ng RNA) | | Pathology | B1799(copy/ng RNA) |
| 1 | normal | 5196.77 | 21 | moderate | 3865.66 |
| 2 | normal | 2813.08 | 22 | moderate | 3931.79 |
| 3 | normal | 4443.58 | 23 | moderate | 6614.39 |
| 4 | normal | 4115.51 | 24 | moderate | 6352.02 |
| 5 | normal | 3890.60 | 25 | moderate | 15627.77 |
| 6 | normal | 1897.45 | 26 | moderate | 4516.00 |
| 7 | normal | 2163.33 | 27 | moderate | 8289.22 |
| 8 | normal | 154.31 | 28 | moderate | 17001.41 |
| 9 | normal | 3542.13 | 29 | moderate | 5502.07 |
| 10 | normal | 1647.13 | 30 | moderate | 21914.55 |
| 11 | mild | 1176.89 | 31 | severe | 2645.44 |
| 12 | mild | 10222.95 | 32 | severe | 3492.70 |
| 13 | mild | 15579.36 | 33 | severe | 5810.25 |
| 14 | mild | 18515.82 | 34 | severe | 13260.42 |
| 15 | mild | 3200.39 | 35 | severe | 736.77 |
| 16 | mild | 1862.32 | 36 | severe | 6562.17 |
| 17 | mild | 5031.60 | 37 | severe | 11227.19 |
| 18 | mild | 5999.48 | 38 | severe | 5974.82 |
| 19 | mild | 5382.48 | 39 | severe | 3249.61 |
| 20 | mild | 1019.33 | 40 | severe | 609.08 |

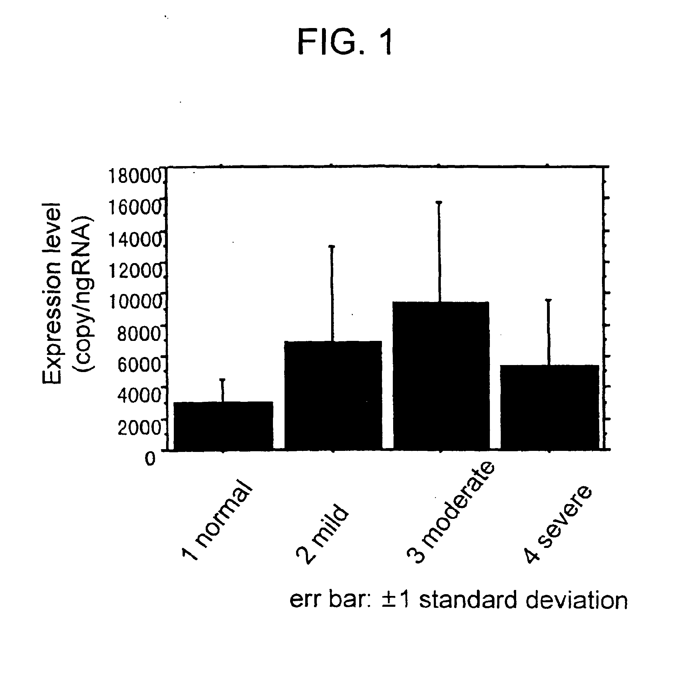

Specifically, the expression level was high in patients with moderate pathology compared to healthy subjects and patients with mild pathology. More specifically, analysis of variance was performed among four groups consisting of groups of atopic dermatitis classified into groups of mild, moderate, and severe pathologies, and the healthy subject group , indicating that there are significant differences (p<0.05) among these four groups. Comparison of the differences among the groups by post hoc test according to the PLSD method of Fisher, showed the significant difference and bias between the groups as follows. Expression in each group is shown in Fig. 1.
healthy subject < mild pathology subject
   (bias p<0.1)
healthy subject < moderate pathology subject
   (significant difference p<0.01)
severe pathology subject < moderate pathology subject
   (bias p<0.1)

As demonstrated above, the expression level was revealed to be higher in atopic dermatitis patients, especially in patients with moderate pathology, compared to normal healthy subjects.

### [Example 5]

### Expression levels in various immunocytes

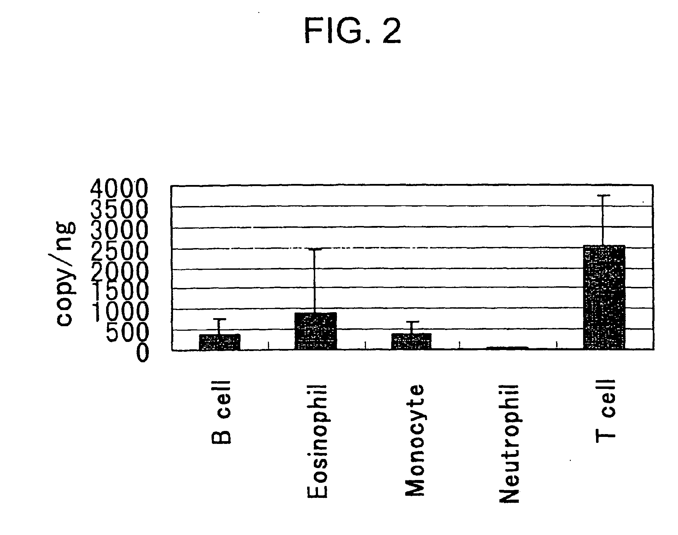

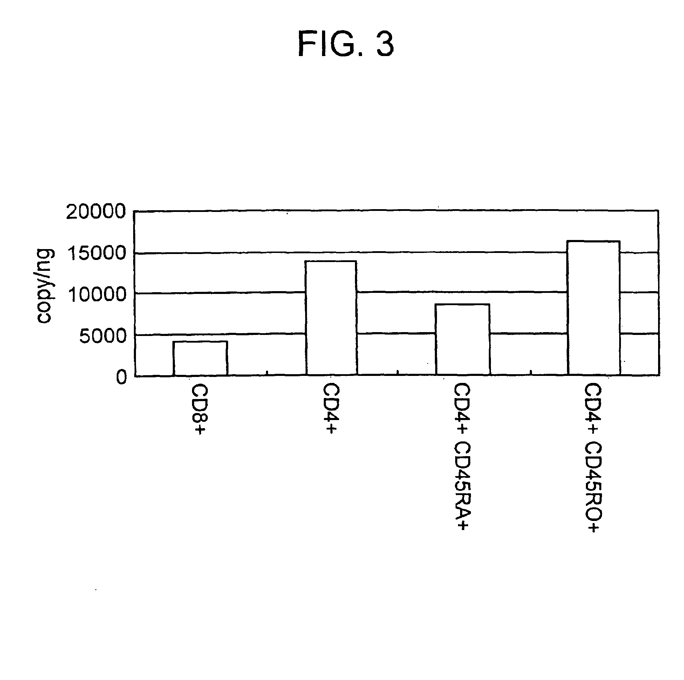

Expression levels in T cells, B cells, eosinophils, monocytes, and neutrophils were measured in RNA samples derived from 5 subjects. The result revealed that the expression level was highest in T cells (Fig. 2). Among the T cells, the expression was higher in T cells with the phenotypes of CD4⁺ and CD45RO⁺, i.e. , memory T cells (Fig. 3) .

### [Example 6]

### Changes in expression due to T cell activation

Once mature T cells are stimulated with antigen, a further stimulation with the antigen is known to cause apoptosis which is called activated cell death of T cells. Activated cell death of T cells is suggested to be involved in the control of immunological tolerance and immune response in periphery (Yili Yang et al., J. Exp, Med. 181: 1673-1682, 1995). To examine the role of B1799 in these controls, the expression changes of B1799 due to activated cell death of T cells and cell death caused by glucocorticoid were measured. Peripheral blood-derived T cells were cultured in 5%FCS-containing RPMI1640 medium supplemented with 1 mM sodium pyruvate, 2 mM L-glutamine, 100 u/ml penicillin, and 100 µg/ml streptomycin, further with 200 u/ml IL-2 (Imunace®, Shionogi & Co., Ltd.) under 5% carbon dioxide and 95% humidity at 37°C. First, CD3+ cells isolated from periphery were cultured for 5 days on plates coated with an anti-CD3 antibody (OKT3, Janssen-Kyowa Co., LTD), followed by cultivation for 3 days without the anti-CD3 antibody. The obtained T cells were cultured again with stimulus on the anti-CD3 antibody coated plates to measure activated cell death. Cells are collected over the course of time, and RNA was extracted according to conventional methods for quantitative PCR. For control without stimulus, plates without an anti-CD3 antibody were used.

Further, dexamethasone was added to unstimulated control as glucocorticoid that induces apoptosis of T cells (Kofler R., Histochem. Cell Biol., 2000 Jul; 114(1): 1-7), and its influence was examined.

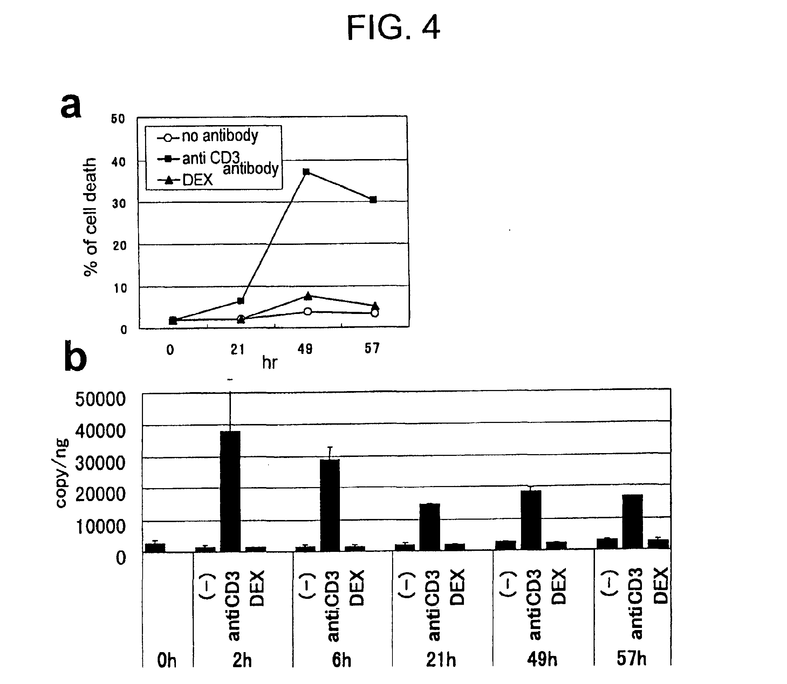

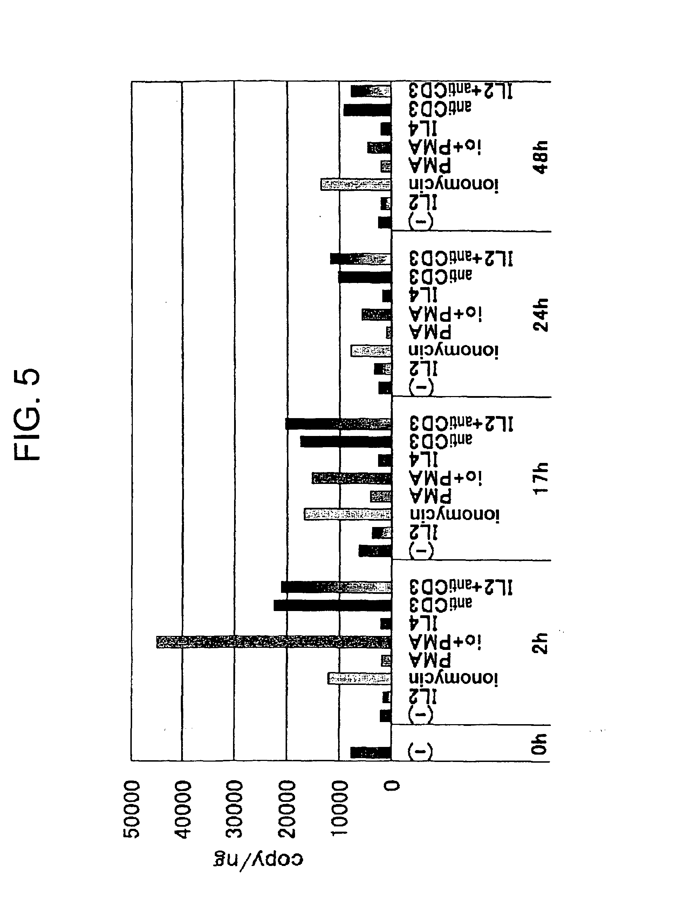

As depicted in Fig. 4a, obvious cell death was induced 49 hours after the second stimulation via the T cell receptor on human periphery-derived T cells using anti-CD3 antibodies. B1799 was markedly induced 2 hours after the stimulation during the T cell activation process. In contrast, no notable cell death of peripheral T cells or changes in the expression of B1799 was observed in the presence of dexamethasone (Fig. 4b "DEX"). Through the stimulation of T cells by various reagents in similar experiments, the expression of B1799 was shown to be markedly induced by stimulation with 1 µg/ml of ionomycin (Sigma) , calcium ionophore, alone (described as "ionomycin" in the Figure), or with 25 ng/ml of phorbol 12-myristate 13-acetate (PMA, Sigma) (described as "io+PMA" in the Figure) (Fig. 5) , in addition to anti-CD3 antibodies. The above results show that the expression of B1799 was enhanced together with the activation of the T cell. Furthermore, B1799 was suggested to be involved in some way to subsequent activated cell death.

### [Example 7]

### Analysis of amino acid sequence structure

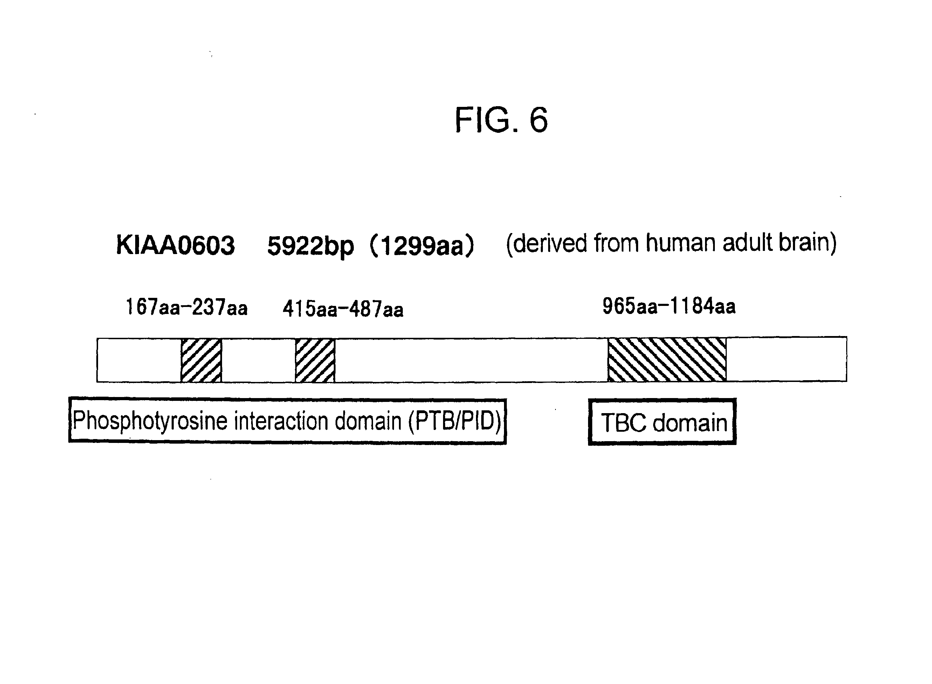

The structure of the protein encoded by B1799 is shown in Fig. 6. The protein has, at its N-terminus region, two phosphotyrosine interaction domain (PID) that is involved in the inter-protein binding in the phosphorylated tyrosine binding region. Furthermore, the protein has a TBC domain at its C-terminus.

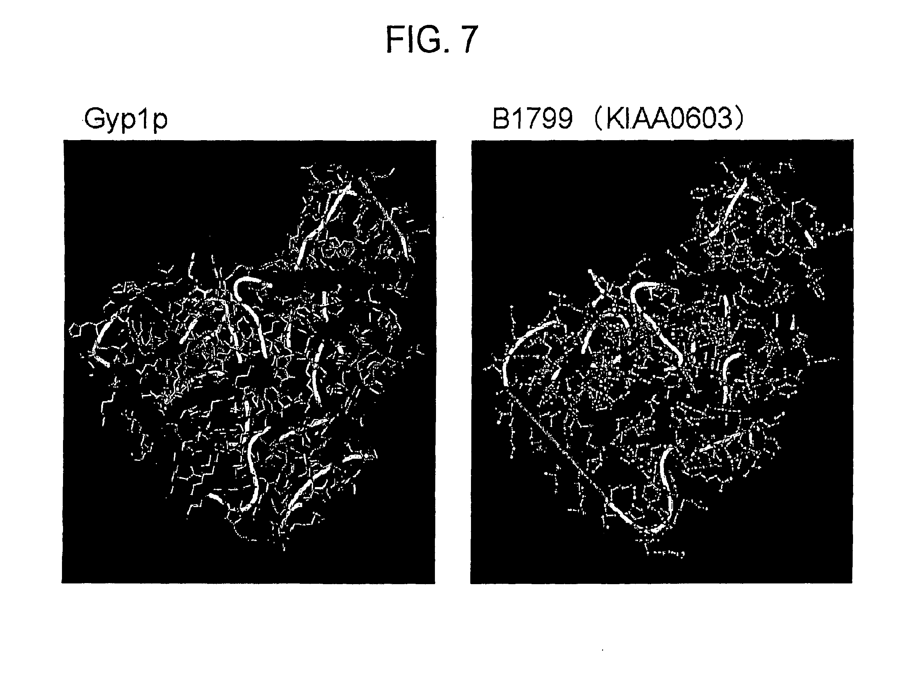

BLAST search revealed that the region of amino acids from the position 886 to 1237 , which contains the TBC domain, shows a homology of 31% to human rab6 GTPase activating protein (Cuif M.H. et al., EMBO J. :18(7) 1772-82, 1999) (ACCESSION NP_036329). Further, BLAST search showed that the amino acid region from the position 853 to 1107 has a homology of 21% to Gyp1p protein (ACCESSION NP_014713), yeast GTPase activating protein (GAP). As demonstrated in Fig. 7 , B1799 protein was predicted to have a very similar tertiary structure to the GTPase activating site structure of Gyp1, whose crystal structure had been determined (Rak A. et al., EMBO J. 19: 5105-5113, 2000). Arg973 residue of B1799 protein corresponds to Arg343 residue of Gyp1p, which is essential as the GTPase activating site. Therefore, there is a high possibility that the B1799 protein is a GTPase activating protein, and the Arg973 residue was predicted to play an important role in its activity.

The GTP binding form of the GTP binding protein, which protein has an important role in the signal transduction, is the activated form in the signal transduction and changes to the inactivated form when the bound GTP is converted to GDP. The GTP binding protein itself has the GTPase activity and GTPase activating proteins have the function to enhance the GTPase activity. GTPase activating proteins help the conversion of GTP binding proteins to the inactivated form, thereby regulating signal transduction.

As described above, B1799 would play an important role in signal transduction where the T cell gets activated by regulating the activity of the GTP binding protein.

Furthermore, throughout the whole sequence, the amino acid sequence encoded by B1799 showed a high homology to mouse Tbc1. Tbc1 was discovered as a gene whose expression changes during mast cell differentiation, and is suggested to be involved in cell differentiation and cell cycle suppression (Paul M. Richardson and Leonard I. Zon., Oncogene 11: 1139-1148, 1995). Thus, similarly to Tbc1, B1799 may be involved in cell differentiation and cell cycle suppression.

### Industrial Applicability

The present invention provided a gene that shows a difference in expression between healthy subjects and patients with allergic diseases. Using the expression of the gene of the present invention as a marker, it became possible to test for allergic diseases and screen for candidate therapeutic compounds. The test methods of the present invention can be readily conducted by measuring the expression level of the marker gene and can quickly find the pathological state of allergic reaction. Additionally, according to this invention, the methods of testing for allergies have low invasiveness towards patients since analysis of the expression level can be carried out using biological samples, like peripheral blood leukocytes. Furthermore, highly sensitive measurements in gene expression analysis are possible using small sample amounts. Year after year, high throughput methods and cost reduction are progressing in gene analysis technology. Therefore, in the near future, the methods of testing for allergies of this invention are expected to become important bed-side diagnostic methods. In this sense, the diagnostic value of these pathology-related genes is high. Furthermore, the screening methods of the present invention are expected to be applied to development of novel therapeutic agents for allergic diseases.

## Claims

1. A method of testing for an allergic disease, the method comprising the steps of:
(a) measuring the expression level of a marker gene in a biological sample from a test subject;
(b) comparing the expression level with that of the marker gene in a biological sample from a healthy subject; and
(c) judging the test subject to have an allergic disease when the expression level of the marker gene in the biological sample from the test subject is found to be significantly elevated. wherein the marker gene is B1799 gene.

2. The testing method according to claim 1, wherein the allergic disease is atopic dermatitis.

3. The testing method according to claim 1, wherein the gene expression level is measured via cDNA PCR.

4. The testing method according to claim 1, wherein the gene expression level is measured by detecting a protein encoded by the gene.

5. The testing method according to claim 1, wherein the biological sample contains peripheral blood T cells.

6. A reagent for diagnosis of an allergic disease, said reagent comprising a polynucleotide that comprises at least 15 continuous nucleotide sequence of B1799 gene or a complementary sequence thereof;

7. A reagent for testing for an allergic disease, said reagent comprising an antibody that binds to a polypeptide consisting of the amino acid sequence encoded by B1799 gene;

8. A method for screening a therapeutic agent for an allergic disease, wherein said method comprises the steps of:
(a) contacting a candidate compound with a cell expressing a marker gene;
(b) measuring the expression level of said marker gene; and
(c) selecting a compound which decreases the expression level of the marker gene as compared to a control where said candidate compound has not been contacted,
wherein the marker gene is B1799 gene.

9. The method according to claim 8, wherein the cell is T cell.

10. A method of screening for a therapeutic agent for an allergic disease, the method comprising the steps of:
(a) administering a candidate compound to a test animal;
(b) measuring the expression level of a marker gene in leukocytes of the test animal; and
(c) selecting a compound which decreases the expression level of the marker gene compared to a control where the candidate compound has not been administered,
wherein the marker gene is B1799 gene.

11. A method of screening for a therapeutic agent for an allergic disease, the method comprising the steps of:
(a) contacting a candidate compound with cells containing a reporter gene linked under the control of transcriptional regulatory region of a marker gene;
(b) measuring the expression level of the reporter gene; and
(c) selecting a compound which decreases the expression level of the reporter gene compared to a control where the candidate compound has not been contacted,
wherein the marker gene is B1799 gene.

12. A method of screening for a therapeutic agent for an allergic disease, the method comprising the steps of:
(a) contacting a candidate compound with a protein encoded by a marker gene or a gene functionally equivalent thereto;
(b) measuring the activity of the protein; and
(c) selecting a compound which decreases the activity of the protein compared to a control where the candidate compound has not been contacted,
wherein the marker gene is B1799 gene.

13. A therapeutic agent for an allergic disease comprising as the main ingredient a compound obtainable by the screening method according to any one of claim 8, 10, 11, and 12.

14. A therapeutic agent for an allergic disease, which comprises as a main ingredient an antisense DNA that contains a sequence complementary to a sequence comprising at least 15 continuous nucleotides of the sense strand sequence of a marker gene,
wherein the marker gene is B1799 gene.

15. A therapeutic agent for an allergic disease, which comprises as a main ingredient an antibody which binds to a protein encoded by a marker gene, wherein the marker gene is B1799 gene.

16. An animal model of allergic disease, wherein said animal is a transgenic nonhuman vertebrate, in which the expression level of a marker gene, or a gene functionally equivalent thereto, has been increased in T cells, wherein the marker gene is B1799 gene.

17. A kit for screening for a therapeutic agent for an allergic disease, the kit comprising a polynucleotide comprising at least 15 continuous nucleotide sequence of a marker gene or its complementary sequence, wherein the marker gene is B1799 gene.

18. A kit for screening for a therapeutic agent for an allergic disease, the kit comprising an antibody which binds to a polypeptide consisting of an amino acid sequence encoded by a marker gene and cells expressing the marker gene, wherein the marker gene is B1799 gene.
